# EUROPEAN PATENT APPLICATION

(11) **EP 4 215 542 A1**
(43) Date of publication of application: **26.07.2023**
(21) Application number: 21869476.8
(22) Date of filing: 17.09.2021
(51) Int. Cl.: C07K 14/55, A61K 38/20, A61K 47/64, A61K 47/68, A61P 35/00, A61P 43/00, C07K 14/76, C07K 16/00, C07K 19/00, C12N 15/26

(54) **IL-2 MUTANT PROTEIN AND MEDICINE CONTAINING SAME**

(30) Priority: 18.09.2020 JP 2020157900
(71) Applicant: Miyagi Prefectural Hospital Organization, Natori-shi, Miyagi 981-1239 (JP)
(72) Inventor: TANAKA, Nobuyuki, Medeshima-Shiote, Natori-shi, Miyagi 981-1293 (JP)
(74) Representative: Winter, Brandl - Partnerschaft mbB
(86) International application number: PCT/JP2021/034438
(87) International publication number: WO 2022/059794

(57) **Abstract**

Provided is an IL-2 mutein having an amino acid sequence set forth in SEQ ID NO: 3 or an amino acid sequence which has at least 95% sequence identity to the amino acid sequence of SEQ ID NO: 3 and in which amino acids at position 35, position 38, position 42 or position 43, and position 45 are all A's.

## Description

### Technical Field

### [Cross-reference to Related Application]

This application claims priority from Japanese Patent Application No. 2020-157900, filed on September 18, 2020, the entire disclosure of which is incorporated herein by reference. The present invention relates to an IL-2 mutein and a pharmaceutical including the IL-2 mutein.

### Background Art

It is useful to stimulate the immune system of a mammal towards an adaptive immune response, which has evolved to clear pathogens that have entered the body. An important population of immune cells that are thereby activated are CD8+ effector T cells (cytotoxic lymphocytes). IL-2 is known to stimulate a variety of immune cells, including monocytes, NK cells, and T cells. IL-2 is used in the clinic to stimulate a cell-mediated immune response, and is approved by the FDA for standard therapy in patients with metastatic melanoma or metastatic kidney cancer (e.g., aldesleukin (Chiron) (also known as Proleukin (trademark))).

The repertoire of T cells involved in a cell-mediated adaptive immune response include CD8+ memory T cells, CD8+ effector T cells, and regulatory T cells (Tregs). These Tregs play an important role in the adaptive immune program by dampening the activity of effector T cells and memory T cells. It has been observed, however, that IL-2 also activates the Treg subset of T cells, which then can act to suppress CD8+ T cells, or to tolerize other T cells. Thus, IL-2 is involved in both the activation of the adaptive immune response and its attenuation.

Treg cells are characterized by the expression of CD4 and the transcription factor FoxP3, which in turn activates the expression of CD25, the α-subunit of the IL-2 receptor complex (CD4+CD25+ cells). Thus, CD25 is constitutively expressed in Treg cells. Association of CD25 with the signaling components of the IL-2 receptor complex (the β-subunit CD122 and the γ-subunit CD132) converts the intermediate-affinity IL-2 receptor complex into a high-affinity IL-2 receptor complex. IL-2 activation of Treg cells occurs through a signaling pathway relayed by the high-affinity IL-2 receptor complex.

High level CD25 expression is a characteristic of activated T cells, making these cells responsive to IL-2 via the high-affinity IL-2 receptor complex. Therapies based on the blockade of CD25 have been developed with the rationale that the blockade of CD25 will inhibit IL-2 mediated signaling in activated T cells and show immunosuppressive effects. Anti-CD25 antibodies, such as daclizumab (Roche) (also known as Zenapax (trademark)) and basiliximab (Novartis) (also known as Simulect (trademark)), have been approved by the FDA for the prevention of acute organ rejection following kidney transplantation. Because of the dual role of IL-2, there remains a need in the art to provide more efficacious IL-2 mediated therapies.

Immune cells involved in a cell-mediated immune response include NK cells having an NK1.1+ phenotype. NK cells play an important role in the adaptive immune program. It has been observed, however, that IL-2 also activates the Treg subset of T cells, which then can act to suppress CD8+ T cells, or to tolerize other T cells. Thus, IL-2 is involved in both the activation of the adaptive immune response and its attenuation.

High level CD25 expression is a characteristic of activated T cells, making these cells responsive to IL-2 via the high-affinity IL-2 receptor complex. Therapies based on the blockade of CD25 have been developed with the rationale that the blockade of CD25 will inhibit IL-2 mediated signaling in activated T cells and show immunosuppressive effects. Anti-CD25 antibodies, such as daclizumab (Roche) (also known as Zenapax (trademark)) and basiliximab (Novartis) (also known as Simulect (trademark)), have been approved by the FDA for the prevention of acute organ rejection following kidney transplantation. Because of the dual role of IL-2, there remains a need in the art to provide more efficacious IL-2 mediated therapies.

### Citation List

### Patent Literature

PTL 1: WO 2020/069398 A1
PTL 2: WO 2018/215936 A1
PTL 3: WO 2018/215938 A1
PTL 4: WO 2014/028748 A1
PTL 5: JP 2015-229676 A

### Non-patent Literature

NPL 1: Proc. Natl. Acad. Sci. U.S.A. (2006) 103 p. 2788-93
NPL 2: Adv Drug Deliv Rev. 2013 Oct 15; 65(10): 1357-1369.

### Summary of Invention

### Technical Problem

An object of the present invention is to provide an IL-2 mutant having attenuated binding capability for an IL-2 receptor α while maintaining binding capability for IL-2 receptors β and γ.

### Solution to Problem

Under such circumstances, the inventor of the present invention has made extensive investigations, and as a result, has found that, when amino acids at position 35, position 38, position 42 (or position 43), and position 45 of mature IL-2 are all substituted with A's, the binding capability for the IL-2 receptor α is attenuated while the binding capability for the IL-2 receptors β and γ is maintained. The present invention is based on such novel finding. Accordingly, the present invention provides the following items:
Item 1. An IL-2 mutein, including: an amino acid sequence set forth in SEQ ID NO: 3; or an amino acid sequence which has at least 95% sequence identity to the amino acid sequence of SEQ ID NO: 3 and in which amino acids at position 35, position 38, position 42 or position 43, and position 45 are all A's.
Item 2. The IL-2 mutein according to Item 1, wherein the IL-2 mutein is a fusion protein with albumin and/or an immunoglobulin.
Item 3. The IL-2 mutein according to Item 2, wherein an immunoglobulin portion of the fusion protein is an Fc portion of an antibody.
Item 4. The IL-2 mutein according to Item 2, wherein the IL-2 mutein is albumin-IL-2, IL-2-albumin, albumin-IL-2-immunoglobulin, immunoglobulin-IL-2-albumin, albumin-immunoglobulin-IL-2-albumin, or albumin-IL-2-immunoglobulin-albumin.
Item 5. The IL-2 mutein according to any one of Items 1 to 4, wherein the IL-2 mutein has an ability to activate TPA-Mat cells expressing IL-2 receptors β and γ, and has reduced binding capability for an IL-2 receptor α as compared to an IL-2 mutein formed of an amino acid sequence set forth in SEQ ID NO: 2.
Item 6. A pharmaceutical, including the IL-2 mutein of any one of Items 1 to 5.
Item 7. The pharmaceutical according to Item 6, wherein the pharmaceutical is for preventing or treating cancer.
Item 8. A method of preventing or treating cancer, including administering an effective dose of the IL-2 mutein of any one of Items 1 to 5 to a subject in need thereof.
Item 9. A use of the IL-2 mutein of any one of Items 1 to 5, for producing a preventive or therapeutic agent for cancer.

### Advantageous Effects of Invention

According to the present invention, the IL-2 mutant having attenuated binding capability for the IL-2 receptor α while maintaining the binding capability for the IL-2 receptors β and γ can be provided. Accordingly, the use of the IL-2 mutant of the present invention can enhance the efficacy of IL-2 therapy by suppressing the proliferation of Tregs (CD4+CD25+) while retaining an ability to cause CD8+ T cells and NK1.1+ cells to proliferate.

### Brief Description of Drawings

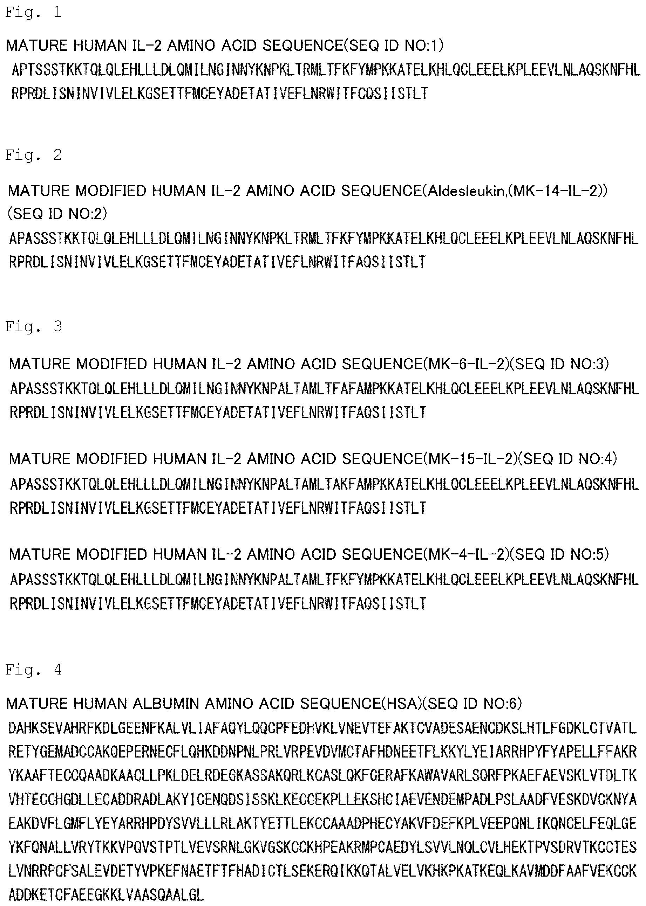

Fig. 1 shows a mature human IL-2 amino acid sequence (SEQ ID NO: 1).
Fig. 2 shows a mature stable mutant human IL-2 amino acid sequence (SEQ ID NO: 2).
Fig. 3 shows mature stable IL-2 receptor α-subunit (CD25) non-binding human IL-2 amino acid sequences (MK-6 (SEQ ID NO: 3), MK-15 (SEQ ID NO: 4), and a mature stable mutant human IL-2 amino acid sequence MK-4 (SEQ ID NO: 5))).
Fig. 4 shows a mature human albumin amino acid sequence (SEQ ID NO: 6).
Fig. 5 shows a mature human albumin-MK-6-IL-2 amino acid sequence (HSA-3×G4S-MK-6) (SEQ ID NO: 7), a mature human albumin-MK-15-IL-2 amino acid sequence (HSA-3×G4S-MK-15) (SEQ ID NO: 8), a mature human albumin-3×(EAAAK)-MK-6-IL-2 amino acid sequence (HSA-3×(EAAAK)-MK-6) (SEQ ID NO: 9), and a mature human albumin-3×(EAAAK)-MK-15-IL-2 amino acid sequence (HSA-3×(EAAAK)-MK-6) (SEQ ID NO: 10).
Fig. 6 shows an amino acid sequence of a mouse albumin protein (SEQ ID NO: 11), an amino acid sequence of a mature MK-6 mouse albumin fusion protein (SEQ ID NO: 12), an amino acid sequence of a mature MK-14 mouse albumin fusion protein (SEQ ID NO: 13), and an amino acid sequence of a mature MK-15 mouse albumin fusion protein (SEQ ID NO: 14).

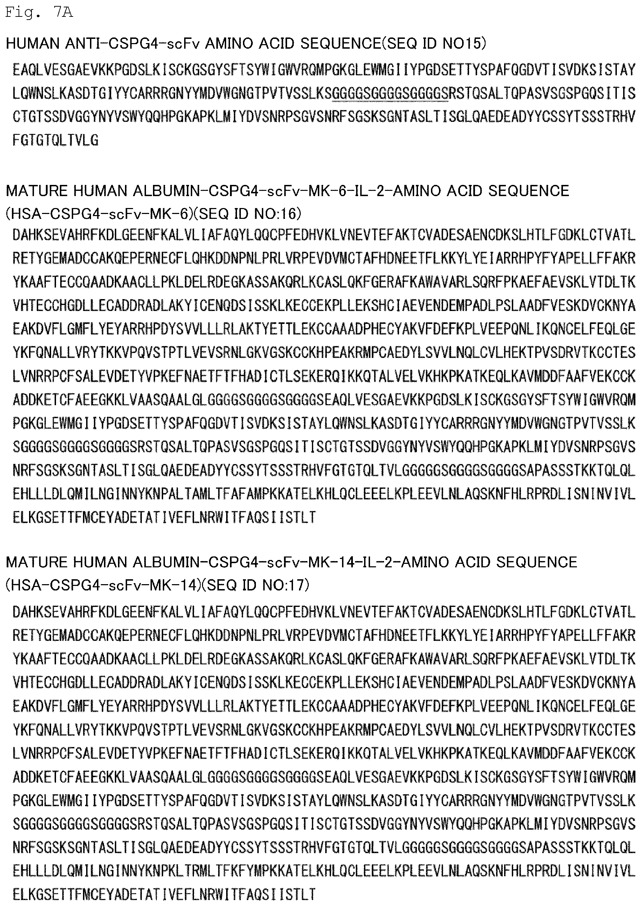

Fig. 7A shows a human anti-CSPG4-scFv amino acid sequence (SEQ ID NO: 15), a mature human albumin-CSPG4-scFv-MK-6-IL-2-amino acid sequence (HSA-CSPG4-scFv-MK-6) (SEQ ID NO: 16), and a mature human albumin-CSPG4-scFv-MK-14-IL-2-amino acid sequence (HSA-CSPG4-scFv-MK-14) (SEQ ID NO: 17).

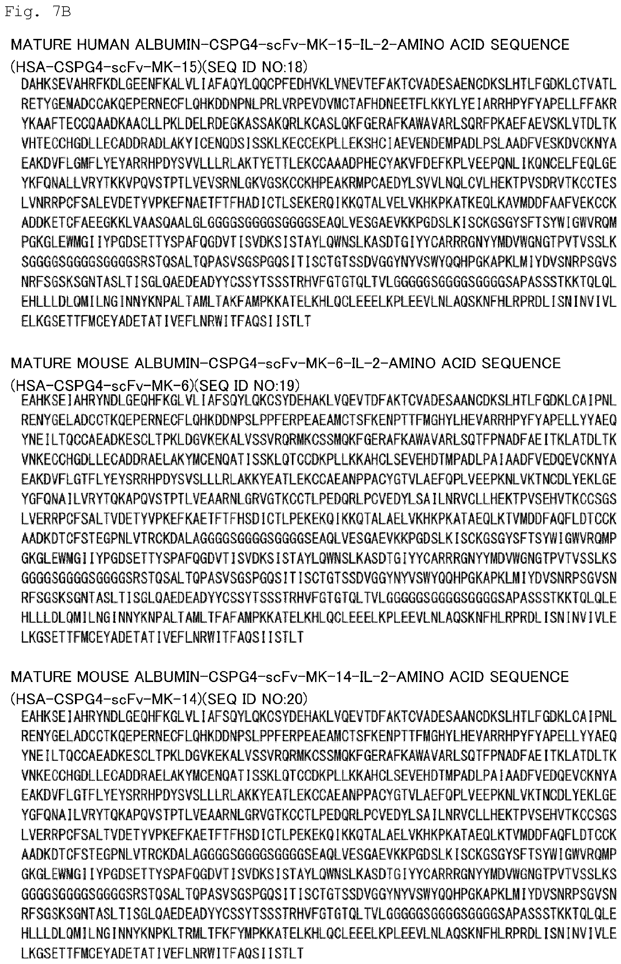

Fig. 7B shows a mature human albumin-CSPG4-scFv-MK-15-IL-2-amino acid sequence (HSA-CSPG4-scFv-MK-15) (SEQ ID NO: 18), a mature mouse albumin-CSPG4-scFv-MK-6-IL-2-amino acid sequence (HSA-CSPG4-scFv-MK-6) (SEQ ID NO: 19), and a mature mouse albumin-CSPG4-scFv-MK-14-IL-2-amino acid sequence (HSA-CSPG4-scFv-MK-14) (SEQ ID NO: 20).
Fig. 8 shows linker amino acid sequences (SEQ ID NOS: 21 and 25) that link mature MK-6 and albumin to each other.
Fig. 9 (top) includes a schematic view of binding between IL-2 and an IL-2 receptor αβγ complex. Fig. 9 (bottom) includes a three-dimensional structural schematic view for binding between IL-2 and the IL-2 receptor αβγ complex, and binding of IL-2.
Figs. 10 show IL-2 receptor α binding capability on cells (on-cell receptor binding).
Fig. 11 shows IL-2 receptor α binding capability on cells (on-cell receptor binding).
Fig. 12 shows human CSPG4 binding capability on cells (on-cell receptor binding).
Fig. 13 shows IL-2-dependent cell proliferation of mouse and human lymphocytes with IL-2, MK-14, and MK-6.
Fig. 14 shows IL-2-dependent cell proliferation of mouse and human lymphocytes with MK-4, MK-6, MK-14, and MK-15.
Fig. 15 shows IL-2-dependent cell proliferation of mouse and human lymphocytes with MK-6, HSA-3×G4S-MK-6, and HSA-3×(EAAAK)-MK-6.
Fig. 16 shows IL-2-dependent cell proliferation of mouse and human lymphocytes with MK-6, HSA-CSPG4-scFv-3×G4S-MK-6, and HSA-CSPG4-scFv-3×G4S-MK-14.
Fig. 17 shows IL-2-dependent Treg cell proliferation.
Fig. 18 shows effects on peripheral immune system cell counts in mice (BALB/c) injected with IL-2 (MK-6-IL-2 or MK-14-IL-2) .
Fig. 19 shows effects on body weight, and liver and spleen weights in mice (BALB/c) intravenously injected with IL-2 (MK-6-IL-2 or MK-14-IL-2) once daily for 4 consecutive days.
Fig. 20 shows the effect of IL-2 (MK-6-IL-2 or MK-14-IL-2) on cancer-bearing mice (BALB/c) subcutaneously implanted with CT26 colon cancer cells.
Figs. 21 show FACS analysis of intratumoral lymphocytes (TILs) excised after mice (BALB/c) subcutaneously implanted with CT26 colon cancer cells have been intraperitoneally injected with IL-2 (MK-6-IL-2 or MK-14-IL-2) twice daily for 5 consecutive days.
Fig. 22 shows the effect of IL-2 (MK-6-IL-2 or MK-14-IL-2) on cancer-bearing mice (C57BL/6) subcutaneously implanted with B16F10 melanoma.
Fig. 23 shows FACS analysis of intratumoral lymphocytes (TILs) through IL-2 monotherapy in a B16F10 melanoma-bearing mouse model.
Figs. 24 show the antitumor effect of albumin-fused IL-2 (MSA-IL-2) monotherapy in a CT26 colon cancer-bearing mouse model.
Figs. 25 show changes in IL-2 blood concentration after intraperitoneal injection of mature MK-6-IL-2 and mature MK-6-IL-2-mouse albumin and quantification of intratumoral IL-2 in a CT26 colon cancer-bearing mouse model.

### Description of Embodiments

In the present invention, the terms "protein" and "peptide" are each used in a meaning including an oligopeptide and a polypeptide. In addition, herein, the terms "protein" and "peptide" each encompass both of a protein modified with a glycan or the like and an unmodified protein, unless otherwise stated. The same applies to a protein that is not specified to be a protein.

### IL-2 Mutein

The present invention provides an IL-2 mutein having an amino acid sequence set forth in SEQ ID NO: 3 or an amino acid sequence which has at least 95% sequence identity to the amino acid sequence of SEQ ID NO: 3 and in which amino acids at position 35, position 38, position 42 or position 43, and position 45 are all A's. Accordingly, the present invention provides an IL-2 mutein having: the amino acid sequence set forth in SEQ ID NO: 3; an amino acid sequence which has at least 95% sequence identity to the amino acid sequence of SEQ ID NO: 3 and in which the amino acids at position 35, position 38, position 42, and position 45 are all A's; or an amino acid sequence which has at least 95% sequence identity to the amino acid sequence of SEQ ID NO: 3 and in which the amino acids at position 35, position 38, position 43, and position 45 are all A's. An example of the amino acid sequence which has at least 95% sequence identity to the amino acid sequence of SEQ ID NO: 3 and in which the amino acids at position 35, position 38, position 42, and position 45 are all A's is an amino acid sequence set forth in SEQ ID NO: 4.

In the present invention, the IL-2 mutein having the amino acid sequence set forth in SEQ ID NO: 3 encompasses not only an IL-2 mutein whose amino acid sequence is formed only of the amino acid sequence set forth in SEQ ID NO: 3, but also an IL-2 mutein whose amino acid sequence contains as a part thereof the amino acid sequence set forth in SEQ ID NO: 3. Similarly, in the present invention, the IL-2 mutein having the amino acid sequence which has at least 95% sequence identity to the amino acid sequence of SEQ ID NO: 3 and in which the amino acids at position 35, position 38, position 42 or position 43, and position 45 are all A's encompasses not only an IL-2 mutein whose amino acid sequence is formed only of the amino acid sequence which has at least 95% sequence identity to the amino acid sequence of SEQ ID NO: 3 and in which the amino acids at position 35, position 38, position 42 or position 43, and position 45 are all A's, but also an IL-2 mutein whose amino acid sequence contains as a part thereof the amino acid sequence which has at least 95% sequence identity to the amino acid sequence of SEQ ID NO: 3 and in which the amino acids at position 35, position 38, position 42 or position 43, and position 45 are all A's.

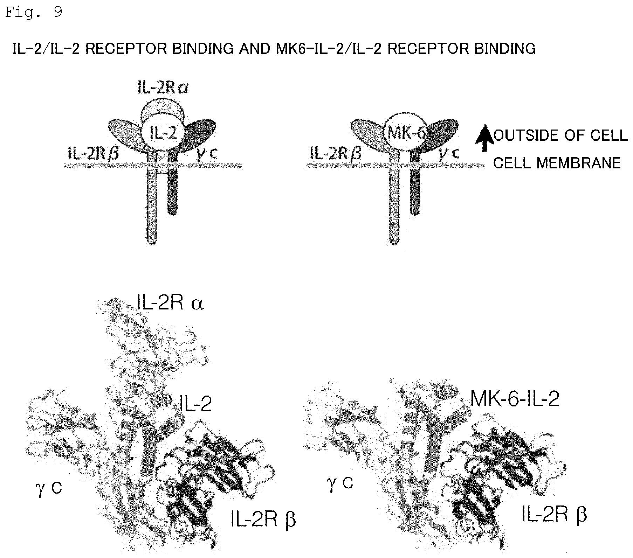

SEQ ID NO: 2 sets forth the amino acid sequence of a mature stable mutant human IL-2. The amino acid sequence set forth in SEQ ID NO: 3 corresponds to an amino acid sequence obtained by substituting all of K at position 35, R at position 38, K at position 43, and Y at position 45 in SEQ ID NO: 2 with A's. In addition, the amino acid sequence set forth in SEQ ID NO: 4 corresponds to an amino acid sequence obtained by substituting all of K at position 35, R at position 38, F at position 42, and Y at position 45 in SEQ ID NO: 2 with A's. Now, outlines of IL-2/IL-2 receptor binding, and binding between MK-6-IL-2 (described in Examples to be described later) according to one typical embodiment of the present invention and the IL-2 receptor are illustrated in Fig. 9. Specifically, Fig. 9 (top) includes a schematic view of binding between IL-2 and an IL-2 receptor αβγ complex. Mature human IL-2 (and mature modified human IL-2) binds to the high-affinity IL-2 receptor αβγ complex. Meanwhile, mature MK-6-IL-2 does not bind to the α-subunit, and binds to an intermediate-affinity IL-2 receptor made up of βγ.

Fig. 9 (bottom) includes a three-dimensional structural schematic view for binding between IL-2 and the IL-2 receptor αβγ complex, and binding of IL-2. The three-dimensional structural schematic view of each protein in Fig. 9 (bottom) was created using known data (2ERJ) from the Protein Data Bank (PDB).

The IL-2 receptor α is widely expressed in vascular endothelial cells, Treg cells, activated T cells, and the like. Mature human IL-2 (and mature modified human IL-2) binds to cells expressing the IL-2 receptor α alone. Consequently, the following plurality of adverse events are caused. Firstly, one of the adverse events is vascular leak syndrome (VLS). Binding to vascular endothelial cells expressing the IL-2 receptor α alone increases vascular permeability, and hence sometimes causes systemic multiple organ edema and multiple organ failure which are lethal. Secondly, another adverse event is the activation of Tregs. Tregs suppress antitumor immunity, and hence induce the growth and metastasis/invasion of cancer.

Three subunits, i.e., α (CD25), β (CD122), and γc (CD132) have been identified for the IL-2 receptor (IL-2R). IL-2Rα serves alone as a low-affinity receptor, a heterodimer of IL-2Rβ and γc serves as an intermediate-affinity receptor, and αβγ serves as a high-affinity receptor. Of those, only the latter two have signaling. Tyrosine kinases JAK1 and JAK3 associate with IL-2Rβ and γc, respectively, to mainly activate STATS. Further, activation of PI3K, Akt, and MAPK systems occurs. IL-2 acts on T cell proliferation. One of its effects is proliferation and activation of CD8⁺ T cells subjected to antigen stimulation. In fact, IL-2 regulates antigen-dependent proliferation of CD8⁺ T cells throughout all differentiation stages: naive, effector, and memory. At least part of the CD8⁺ T cells are called cytotoxic T cells or killer T cells, and are known to kill tumor cells presenting cancer-associated antigens. Further, NK cells express the intermediate-affinity IL-2 receptor made up of βγ, and are proliferated and activated by IL-2 stimulation to exhibit cytotoxic activity including antitumor activity. Meanwhile, IL-2 is required for differentiation of both natural Tregs (nTregs), which are produced in the thymus, and induced Tregs (iTregs), which are produced in the periphery. Treg cells are characterized by constitutively expressing, at a high level, the high-affinity IL-2 receptor IL2Rαβγ made up of the IL2 receptor α-subunit (CD25), the IL2 receptor β-subunit (CD122), and the IL2RG-subunit (CD132), and the proliferation of Treg cells has been shown to be dependent on IL-2. In antitumor immunity, it is known that immunosuppression caused by Treg cells precludes effective functioning of antitumor immunity and induction of activation of antitumor activity, and hence growth or metastasis/invasion of a tumor occurs.

Now, as described in Non-patent Literature 1, of the constituent amino acids of IL-2, ten-odd amino acids are present in the vicinity of a binding portion between IL-2 and the IL-2 receptor α. The inventor of the present invention has conceived of mutating some of those ten-odd amino acids through extremely many investigations made with a view to lowering binding capability between IL-2 and the IL-2 receptor α, and after a great deal of further trial and error, and has arrived at focusing on K at position 35, R at position 38, F at position 42 or K at position 43, and Y at position 45 of IL-2, and arrived at obtaining an IL-2 mutant showing attenuated binding capability for the IL-2 receptor α while maintaining binding capability for the IL-2 receptors β and γ by substituting those amino acids with A's. Accordingly, as described in Examples of the present application, the IL-2 mutein having the amino acid sequence set forth in SEQ ID NO: 3 shows attenuated binding capability for the IL-2 receptor α while maintaining the binding capability for the IL-2 receptors β and γ conceivably because K at position 35, R at position 38, F at position 42 or K at position 43, and Y at position 45, which are brought into contact with the IL-2 receptor α, have all been substituted with A's. Accordingly, the IL-2 mutein of the present invention may have an amino acid sequence having one or several (e.g., 1 to 7, 1 to 6, 1 to 5, 1 to 4, 1 to 3, 1 or 2, or 1) amino acids added, deleted, and/or substituted in the amino acid sequence set forth in SEQ ID NO: 3 so as to conserve all of A's at position 35, position 38, and position 45, and to conserve A at position 43 or have A at position 42. In such embodiment, the IL-2 mutein preferably has the amino acid sequence set forth in SEQ ID NO: 3 or at least 95% sequence identity to the amino acid sequence of SEQ ID NO: 3, more preferably has at least 97% sequence identity thereto, and still more preferably has at least 99% sequence identity thereto. A non-limiting example of the amino acid sequence having amino acids added, deleted, and/or substituted in the amino acid sequence set forth in SEQ ID NO: 3 so as to conserve all of A's at position 35, position 38, and position 45 in the amino acid sequence set forth in SEQ ID NO: 3, and to conserve A at position 43 or have A at position 42 therein is the amino acid sequence set forth in SEQ ID NO: 4. In addition, non-limiting examples of the amino acid sequence having amino acids added, deleted, and/or substituted in the amino acid sequence set forth in SEQ ID NO: 3 while conserving all of A's at position 35, position 38, position 43, and position 45 in the amino acid sequence set forth in SEQ ID NO: 3 include: an amino acid sequence obtained by deleting two amino acids between position 81 and position 84 in SEQ ID NO: 3 and adding DTK; an amino acid sequence obtained by deleting P at position 82 in SEQ ID NO: 3; an amino acid sequence obtained by substituting K at position 49 in SEQ ID NO: 3 with Q; an amino acid sequence obtained by substituting A at position 73 in SEQ ID NO: 3 with T; an amino acid sequence obtained by substituting N at position 119 in SEQ ID NO: 3 with R; and an amino acid sequence obtained by combining at least two of those modifications.

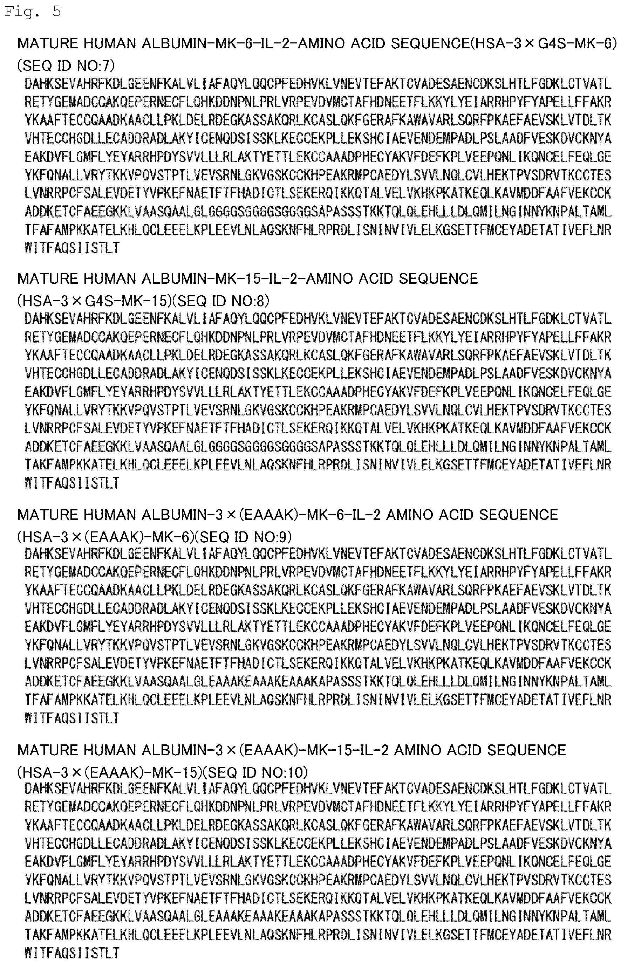

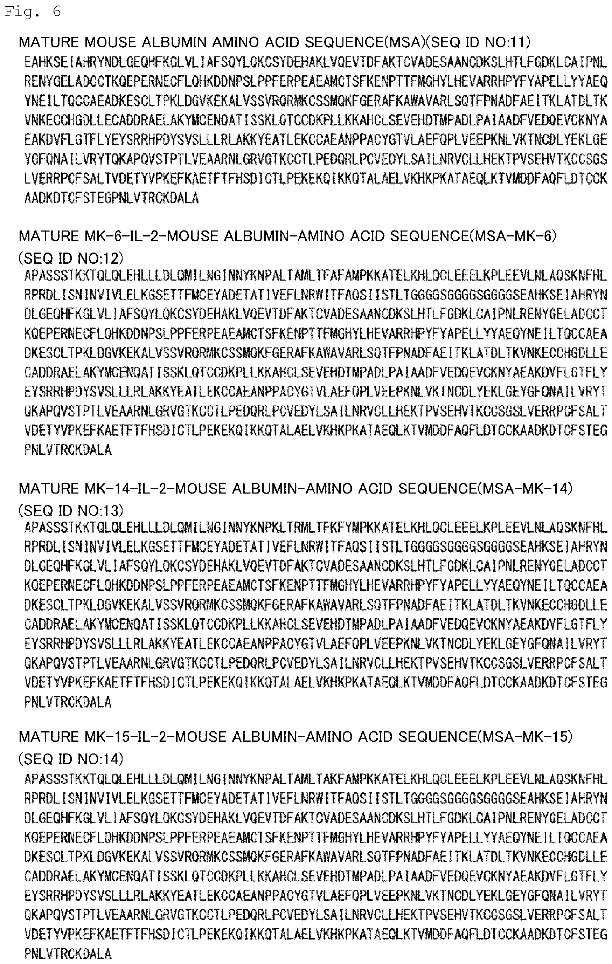

In the present invention, to the extent that the effect of the present invention is obtained, the IL-2 mutein may be a fusion protein in which another amino acid sequence is fused, optionally via a linker, to the amino acid sequence set forth in SEQ ID NO: 3 or the amino acid sequence having one or several amino acids added, deleted, and/or substituted in the amino acid sequence set forth in SEQ ID NO: 3 so as to conserve all of A's at position 35, position 38, and position 45, and to conserve A at position 43 or have A at position 42. In the present invention, of the constituent polypeptides of the fusion protein, a portion formed of the amino acid sequence set forth in SEQ ID NO: 3 or the amino acid sequence having one or several amino acids added, deleted, and/or substituted in the amino acid sequence set forth in SEQ ID NO: 3 so as to conserve all of A's at position 35, position 38, and position 45, and to conserve A at position 43 or have A at position 42 is sometimes referred to simply as "IL-2 mutein portion." For example, fusion of a stable protein having a long half-life in vivo to the IL-2 mutein portion can enhance the in vivo stability of the IL-2 mutein of the present invention, and hence is preferred. For example, in a preferred embodiment of the present invention, the IL-2 mutein may be a fusion protein of: a protein formed of the amino acid sequence set forth in SEQ ID NO: 3 or the amino acid sequence having one or several amino acids added, deleted, and/or substituted in the amino acid sequence set forth in SEQ ID NO: 3 so as to conserve all of A's at position 35, position 38, and position 45, and to conserve A at position 43 or have A at position 42; and albumin and/or an immunoglobulin. An immunoglobulin portion may be (a) an Fc portion of an antibody and (b) an antibody including a variable region (Fab portion) against an antigen against an antigen molecule presented on a tumor cell or in a tumor cell environment. In addition, in the present invention, the term "immunoglobulin portion" is not limited to one having an Fc portion and a Fab portion, and may refer to any fragment to the extent that the fragment has antigen binding capability (e.g., a Fab-portion fragment, a single-chain variable fragment (scFv), or a variable fragment of a heavy chain antibody (e.g., variable domain of heavy chain of heavy chain antibody (VHH))). In such embodiment, examples of the antibody having a variable region include an anti-EGFR antibody Cetuximab, an anti-Her2 antibody Trastuzumab, and an anti-CD20 antibody Rituximab. In addition, examples of such fusion protein include albumin-IL-2, IL-2-albumin, albumin-IL-2-immunoglobulin, immunoglobulin-IL-2-albumin, albumin-immunoglobulin-IL-2-albumin, and albumin-IL-2-immunoglobulin-albumin. The albumin is, for example, albumin (HSA), or a fragment or mutant of albumin. With regard to mutations involved in such embodiment, it is known to a person skilled in the art that functional mutants of human serum albumin having the same plasma half-life prolonging effect as the wild type (truncated and/or amino acid substituted functional mutants) can be designed. For example, domain III of human serum albumin has been shown to bind to FCGRT(FcRn) to a high degree and it is possible to make mutants including only this domain or combinations with other domains, with long half-lives or half-lives that are modified (e.g., Albufuse Flex Technology, Novozymes). Examples of the protein to be fused to the IL-2 mutein portion include proteins derived from mammals such as humans. In addition, the protein to be fused to the IL-2 mutein portion may be a humanized protein or the like. As some embodiments of the present invention, examples of mature human albumin-MK-6-IL-2 amino acid sequences (Fig. 5, SEQ ID NOS: 7 to 10) and examples of mature MK-6-IL-2-mouse albumin-amino acid sequences (Fig. 6, SEQ ID NOS: 12 to 14) are shown in Fig. 5 and Fig. 6, respectively. In addition, a mature mouse albumin amino acid sequence (SEQ ID NO: 11) for constructing those fusion proteins is shown in Fig. 6. In each of those embodiments, the IL-2 mutein portion and the other protein may be bound to each other via a linker. A linker amino acid sequence may be any of a so-called rigid linker and flexible linker. Examples thereof include those described in Non-patent Literature 2, and specific examples thereof include those shown in the following table:

**Table 1**

| Type | Sequence | |
|---|---|---|
| flexible | GGGGS (described as G4S) | SEQ ID NO: 21 |
| flexible | (GGGGS)₃ (described as 3×G4S) | SEQ ID NO: 23 |
| flexible | (GGGGS)ₙ (n=1, 2, 3, 4) | SEQ ID NOS: 21 to 24 |
| flexible | (Gly)₈ | SEQ ID NO: 25 |
| flexible | (Gly)₆ | SEQ ID NO: 26 |
| rigid | (EAAAK)₃ | SEQ ID NO: 27 |
| rigid | (EAAK)ₙ (n=1 to 3) | SEQ ID NOS: 28 to 30 |
| rigid | A(EAAAK)₄ALEA(EAAAK)₄A | SEQ ID NO: 31 |
| rigid | AEAAAKEAAAKA | SEQ ID NO: 32 |
| rigid | PAPAP | SEQ ID NO: 33 |
| rigid | (AP)ₙ (10aa to 34aa) | |

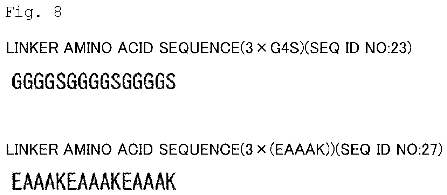

More specifically, in a preferred embodiment, examples of the linker amino acid sequence include: an amino acid sequence set forth in SEQ ID NO: 23 or 27 in Fig. 8; and an amino acid sequence having one or several (e.g., 1 to 7, 1 to 6, 1 to 5, 1 to 4, 1 to 3, 1 or 2, or 1) amino acids added, deleted, and/or substituted in the amino acid sequence set forth in SEQ ID NO: 23 or 27. The amino acid sequence set forth in SEQ ID NO: 23 or a modified sequence thereof is useful because GGGGS or a repeated sequence of GGGGS (e.g., GGGGS GGGGS GGGGS) has the characteristic of having flexibility, and can keep the characteristics of each protein fused. In addition, the amino acid sequence set forth in SEQ ID NO: 27 or a modified sequence thereof is useful because EAAAK or a repeated sequence thereof can reliably keep a distance between two protein domains (separate the domains), and hence allows fusion while keeping a function inherent to a functional domain of each protein.

In the present invention, the IL-2 mutein having the amino acid sequence set forth in SEQ ID NO: 3 also encompasses muteins modified by glycosylation, acetylation, and the like. In the present invention, a mutein modified with a sugar or the like is preferred. In addition, a tag for purification (e.g., a polyhistidine tag or Strep-tag (trademark)) may be added to the N-terminus or C-terminus of the mutant of the present invention by genetic engineering in order to facilitate the purification of the mutant in a purification process to be described later. In addition, the mutant of the present invention may have a secretory signal sequence (also known as a signal peptide or a pre-sequence) bound thereto as an N-terminal extension of the fusion protein in order to direct the fusion protein into the secretory pathway of host cells. A DNA sequence encoding the signal peptide is typically joined to the 5' end of a DNA sequence encoding the fusion protein in the correct reading frame. The signal peptide may be a peptide normally associated with the protein, or may be from a gene encoding another secreted protein or have an artificial secretory sequence.

In a typical embodiment, the IL-2 mutein of the present invention is preferred because of having an ability to activate cells expressing the IL-2 receptors β and γ, and having reduced binding capability for the IL-2 receptor α as compared to an IL-2 mutein formed of the amino acid sequence set forth in SEQ ID NO: 2.

As a method of generating such variant, the variant may be generated by appropriately using a method known per se (e.g., a method described in JP 6640834 B2), for example, constructing a gene cassette obtained by adding the above-mentioned mutations to an IL-2 gene, incorporating the gene cassette into an appropriate vector, and introducing the resultant vector into a host to cause expression. In this case, the expression cassette is joined to an appropriate vector compatible with the host that is being employed. (The vector needs to be able to accommodate the DNA sequence encoding the fusion protein to be expressed.) Suitable host cells include eukaryotic cells and prokaryotic cells that can be easily transformed and can exhibit rapid proliferation in culture medium. Specifically, preferred host cells include prokaryotic cells, such as *E. coli* and *Bacillus subtillus,* and eukaryotic cells, such as animal cells and yeast strains, e.g., S. cerevisiae. Mammalian cells are generally preferred, particularly Expi293F, ExpiCHO, HEK, ExJ558, NSO, SP2-O, or CHO. Other suitable hosts include, for example, insect cells such as Sf9 cells. Stably transformed or transfected cell lines can then be selected. Further, an in vitro transcription-translation system may also be employed as an expression system. In addition, the mutant of the present invention may be produced using a cell-free protein synthesis system. In addition, the resultant mutant of the present invention may be purified appropriately using a method known per se (e.g., filtration, centrifugation, or chromatography).

### Pharmaceutical

In another embodiment, the present invention provides a pharmaceutical containing the above-mentioned IL-2 mutein. By virtue of the effect exhibited by the IL-2 mutein described above, the present invention can provide a pharmaceutical obtained by modifying IL-2 to attenuate its binding to the IL-2 receptor α, and a pharmaceutical obtained by modifying IL-2 to increase its binding to a receptor made up of the IL-2 receptors β and γ. In addition, the pharmaceutical of the present invention may be used for, for example, preventing and/or treating cancer.

In such embodiment, examples of the target cancer include: solid cancers, such as kidney cancer, melanoma, non-small cell lung cancer, colon cancer, hepatocellular carcinoma, and head and neck cancer; and hematological tumors, such as non-Hodgkin lymphoma and acute myeloid leukemia.

In the present invention, the IL-2 mutein serving as the active ingredient of the present invention may be used alone as a pharmaceutical, or may be used as a pharmaceutical composition in combination with any of various pharmaceutically acceptable carriers (e.g., a tonicity agent, a chelating agent, a stabilizing agent, a pH regulator, a preservative, an antioxidant, a solubilizing agent, or a thickening agent).

Examples of the tonicity agent include: sugars, such as glucose, trehalose, lactose, fructose, mannitol, xylitol, and sorbitol; polyhydric alcohols, such as glycerol, polyethylene glycol, and propylene glycol; and inorganic salts, such as sodium chloride, potassium chloride, and calcium chloride.

Examples of the chelating agent include: edentates, such as disodium edetate, calcium disodium edetate, trisodium edetate, tetrasodium edetate, and calcium edetate; ethylenediaminetetraacetate; nitrilotriacetic acid or salts thereof; sodium hexametaphosphate; and citric acid.

An example of the stabilizing agent is sodium hydrogen sulfite.

Examples of the pH regulator include acids, such as hydrochloric acid, carbonic acid, acetic acid, and citric acid, and also include: alkali metal hydroxides, such as sodium hydroxide and potassium hydroxide; alkali metal carbonates or hydrogen carbonates such as sodium carbonate; alkali metal acetates such as sodium acetate; alkali metal citrates such as sodium citrate; and bases such as trometamol.

Examples of the preservative include: sorbic acid; potassium sorbate; parahydroxybenzoates, such as methyl parahydroxybenzoate, ethyl parahydroxybenzoate, propyl parahydroxybenzoate, and butyl parahydroxybenzoate; quaternary ammonium salts, such as chlorhexidine gluconate, benzalkonium chloride, benzethonium chloride, and cetylpyridinium chloride; alkylpolyaminoethylglycine; chlorobutanol; polyquad; polyhexamethylene biguanide; and chlorhexidine.

Examples of the antioxidant include sodium hydrogen sulfite, dried sodium sulfite, sodium pyrosulfite, and concentrated mixed tocopherols.

Examples of the solubilizing agent include sodium benzoate, glycerin, D-sorbitol, glucose, propylene glycol, hydroxypropyl methylcellulose, polyvinylpyrrolidone, macrogol, and D-mannitol.

Examples of the thickening agent include polyethylene glycol, methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and polyvinyl alcohol.

In addition, the above-mentioned pharmaceutical composition may further contain, in addition to the IL-2 mutein, a compound considered to have a preventive or therapeutic action on the disease targeted by the pharmaceutical. When the pharmaceutical is used as an antitumor agent, examples of a compound known to have an antitumor effect include Cetuximab, Trastuzumab, and Rituximab. In the present invention, for example, an immune checkpoint inhibitor, such as Nivolumab, Pembrolizumab, or Ipilimumab, is preferred.

In the embodiment of the pharmaceutical composition, the content of the IL-2 mutein in the composition is not particularly limited, and may be appropriately set within, for example, conditions, such as 90 mass% or more, 70 mass% or more, 50 mass% or more, 30 mass% or more, 10 mass% or more, 5 mass% or more, and 1 mass% or more.

A dosage form is not particularly limited, and examples thereof may include various dosage forms including: orally administered agents, such as a tablet, a pill, a capsule, a powder, a granule, and a syrup; and parenterally administered agents, such as an injection (e.g., intravenous injection, intramuscular injection, or local injection), a gargle, a drop, external preparations (an ointment, a cream, a patch, and an inhalant), and a suppository. Of the dosage forms, for example, orally administered agents (e.g., a tablet, a pill, a capsule, a powder, a granule, and a syrup) and external preparations (e.g., an inhalant, an ointment, a cream, and a patch) are preferred.

In the present invention, the dose of the IL-2 mutein varies depending on, for example, an administration route and the age, body weight, or symptom of a patient, and hence cannot be uniquely defined. However, the dose only needs to be such an amount that a daily dose for adults is generally about 5,000 mg or less, preferably about 1,000 mg or less. The lower limit of the dose of the IL-2 mutein is also not particularly limited, and a daily dose for adults may be appropriately set within the range of generally 0.1 mg or more, preferably 0.5 mg or more. When administered once daily, the IL-2 mutein only needs to be contained in the above-mentioned amount in a single dose. When administered three times daily, the IL-2 mutein only needs to be contained in an amount corresponding to one-third of the above-mentioned amount in a single dose.

The pharmaceutical of the present invention is administered to patients such as mammals. Examples of the mammals include humans, monkeys, mice, rats, rabbits, cats, dogs, pigs, cattle, horses, and sheep. Of those, humans are preferred. Accordingly, in another embodiment, the present invention provides a method of preventing or treating cancer, including administering an effective dose of the IL-2 mutein of any one of Items 1 to 5 to a subject in need thereof.

Specific embodiments of the present invention are described in detail below by way of Examples, but the present invention is not limited to these Examples.

### Examples

### Example 1: IL-2 Receptor α (IL-2Rα) Binding Capability

### Construction of Plasmids:

A plasmid (pcDNA4-IL-2) encoding human IL-2, for encoding the mutein of the present invention, was amplified on the basis of sequence information described in NCBI Reference Sequence: NM_000586.3, by a PCR method using cDNA (PrimeScript (trademark) II 1st strand cDNA Synthesis Kit, Takara Bio Inc., #6210A) derived from mRNA derived from Jurkat cells (#TKG 0209, purchased from the Cell Resource Center for Biomedical Research, Institute of Development, Aging and Cancer, Tohoku University) as a template, and was incorporated into an expression vector for mammalian cells (pcDNA (trademark) 4/myc-HisA Mammalian Expression Vector, Thermo Fisher Scientific K.K.) using an In-fusion reaction (Takara Bio Inc., In-fusion HD Cloning Kit #639648) as a basic step employed to yield DNA encoding the desired fusion. For purification, a protease-cleavable sequence (TEV-cleavable amino acid sequence ENLYFQG), and a Strep-tag (trademark) Strep tag and a polyhistidine tag were fused to the C-terminus of the IL-2 gene. A human IL-2 mutant MK-14 is a product obtained by introducing new mutations into the human IL-2 gene incorporated into the plasmid in the foregoing by a PCR method using synthetic primers (PrimeSTAR (trademark) Max DNA Polymerase, Takara Bio Inc., # R045A), and a plasmid encoding the construct (pcDNA4-MK-14) was constructed. MK-6 and MK-15 each encoding the mutein of the present invention are each a product obtained by introducing new mutations by a PCR method using MK-14 as a template, and plasmids encoding the constructs (pcDNA4-MK-6 and pcDNA4-MK-15) were constructed. In addition, MK-4 is also a product obtained by introducing new mutations by a PCR method using MK-14 as a template, and a plasmid encoding the construct (pcDNA4-MK-4) was constructed.

### Production of IL-2 Muteins:

IL-2 muteins to be used in the following Examples were produced as described below using the plasmids obtained by the above-mentioned methods:
For an IL-2 mutein with a polyhistidine tag, the plasmid encoding the construct was transformed into NEB Stable chemically competent *E. coli* cells (NEB (trademark) #C3040H), a colony was proliferated on an Amp selective agar plate, and the transformant was used for seeding in an LB culture. After having been proliferated overnight, pelletized *E. coli* cells were used for large-scale plasmid preparation (NucleoBond (trademark) Xtra Maxi EF (trademark), MACHEREY-NAGEL GmbH & Co. KG, #740424).

The expression of each IL-2 mutein construct was determined through transient transfection of the plasmid into human embryonic kidney (HEK) cells (Expi293F (trademark), Thermo Fisher Scientific K.K., #A14527) in 250 ml of a suspended culture proliferated in Expi293 (trademark) expression medium (Thermo Fisher Scientific K.K., #A1435101). The Expi293 cells were proliferated at 37°C, 8% CO₂, and a humidity of 80% in a single-use sterile disposable Erlenmeyer flask (flat-bottom 1,000 mL vent filter, Thermo Fisher Scientific K.K.). A shaking speed was 125 rpm in WakenBtech RemoteShake. For each transfection, 250 µg of DNA in 11.25 ml of a transfection medium (Opti-MEM (trademark) I (1x)+GlutaMAX (trademark)-I Reduced Serum Medium, Thermo Fisher Scientific K.K. (trademark) #51985-026) and 675 µl of ExpiFectamine (trademark) 293 Reagent (ExpiFectamine (trademark) 293 Transfection Kit, Life technologies (trademark) #A14525) in 11.83 ml of the transfection medium were used in accordance with the manufacturers' instructions. After 22 hours from the transfection, the culture was supplied with 1.25 ml of Enhancer 1 and 12.5 ml of Enhancer 2 (ExpiFectamine (trademark) 293 Transfection Kit, Thermo Fisher Scientific K.K., #A14525). After about 120 hours from the transfection, the cell culture was harvested through centrifugation for 30 minutes at 4,000 g, and the clarified medium was filtered through 0.22 um Rapid Filter Max Set (Bottle Top & Bottle) 500 ml (Techno Plastic Products AG, #99500, Switzerland) before purification. The resultant was subjected to aseptic filtration through a filter and used for purification of protein expression. The amino acid sequences of MK-6, MK-15, and MK-4 used in the following Examples are set forth in SEQ ID NO: 3, SEQ ID NO: 4, and SEQ ID NO: 5, respectively.

### Purification

The liquid that had been centrifuged and filtered through the 0.22 um filter was applied at low flow rate to a 5 ml HisTrap HP column (GE-Healthcare, #17524802, USA) equilibrated with NPI-20 buffer (50 mM NaH₂PO₄, 300 mM NaCl, 20 mM imidazole, pH 8.0) using an AKTA go chromatography system (GE-Healthcare), and then low-affinity binding impurities were eluted with NPI-20 buffer. The bound fusion protein was eluted with 100% NPI-250 buffer (50 mM NaH₂PO₄, 300 mM NaCl, 250 mM imidazole, pH 8.0), and the main peak was collected using the peak detection option of the Unicorn (trademark) software. The main peak was purified further using Prepacked Disposable PD-10 Columns (GE-Healthcare, #17085101, USA) in 1×D-PBS(-) (Gibco (trademark)). 1 ml fractions were collected and analyzed by reducing SDS-PAGE using SuperSep TM Ace 10 to 20% 13 well (trademark) gel (Fuji-Film Wako, #191-15031). The sample was mixed with 3× sodium dodecyl sulfate (SDS) sample buffer supplemented with 10× reducing agent. The mixture was heated at 95°C for 5 minutes before being loaded for SDS-PAGE. The protein was visualized using QC Colloidal Coomassie stain (BIO-RAD, #1610803) in accordance with the manufacturer's instructions. For the cleavage of the protein with a protease, TEV protease (Applied Biological Materials Inc., #E027) was used. For the quantification of the protein, measurement was performed using Protein Assay BCA Kit (Nacalai Tesque, Inc., #06385-00) in accordance with the manufacturer's instructions.

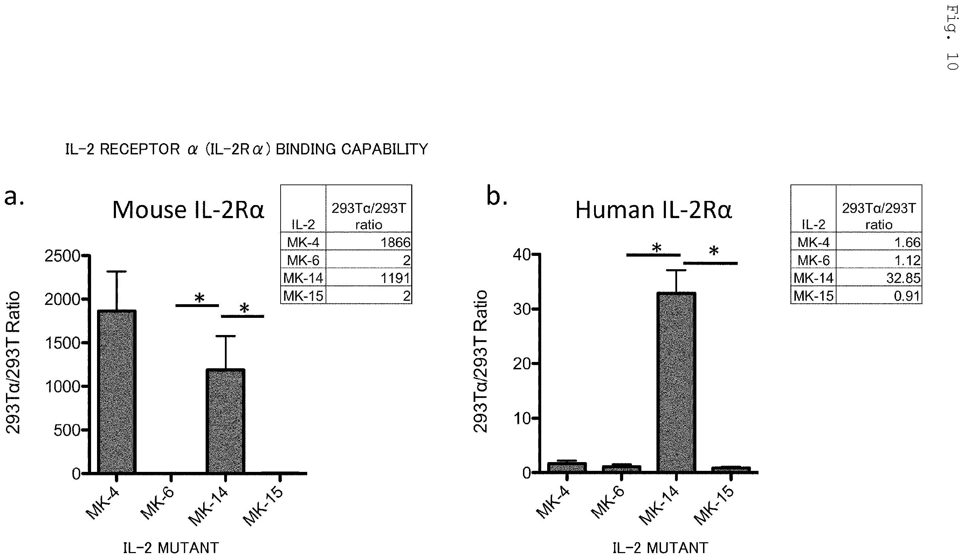

Next, IL-2 receptor α binding capability on cells (on-cell receptor binding) was measured. Specifically, 293T cells were obtained from the RIKEN Cell Bank (RCB2202). Human and mouse IL-2 receptor α-expressing 293T (293Ta) was established as cells stably expressing the IL-2 receptor α in 293T through use of lentiviral vectors (pSIN-EF-RfA2-1-human IL-2Ra and pSIN-EF-RfA2-1-mouse IL-2Ra) constructed respectively with human and mouse IL-2 receptor α-chain genes (NCBI Reference Sequence: NM_000417.2 and NM_008367.3) as cassettes. 293T and human or mouse 293Ta were allowed to adhere to a 24 wel cell culture plate (Techno Plastic Products AG, #92424). After that, a suspension (10 nM) of NanoLuc (trademark) luciferase sequence (Promega Corporation, USA)-fused mature stable mutant human MK-4-IL-2 (MK-4, pcDNA4-MK-4-NLuc, corresponding to a comparative example), mature stable mutant human MK-6-IL-2 (MK-6, pcDNA4-MK-6-NLuc), mature stable mutant human IL-2 (MK-14, pcDNA4-MK-14-NLuc, IL-2 positive control), or mature stable mutant human MK-15-IL-2 (MK-15, pcDNA4-MK-15-NLuc) was added, and the mixture was subjected to a reaction by being left at rest at 4°C for 1 hour. The cells were washed, followed by quantification. The binding capability of mutant IL-2 for the α-subunit is shown as a numerical value for a ratio between amounts bound to 293Ta and 293T (293Ta/293T). The results are shown in Figs. 10. Figs. 10 show mouse and human IL-2 receptor α binding capability. As shown in Fig. 10a, MK-6 and MK-15 did not show binding capability for the mouse IL-2 receptor α. Meanwhile, it is found that MK-4 has high mouse IL-2 receptor α binding capability. In addition, as shown in Fig. 10b, MK-6 and MK-15 did not show binding capability for the human IL-2 receptor α, either.

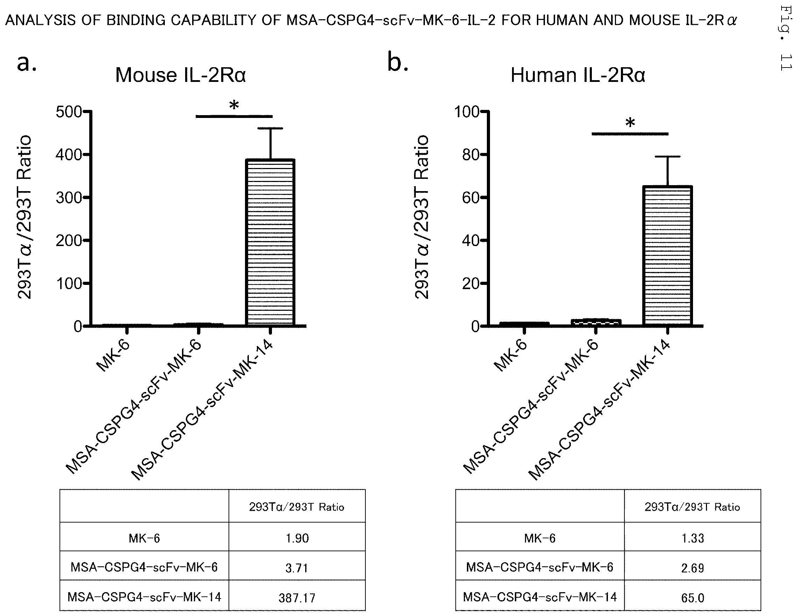

Next, MSA-CSPG4-scFv-MK-14 or MSA-CSPG4-scFv-MK-6 was used as an IL-2 sample to be assayed. MSA-CSPG4-scFv-MK-14 or MSA-CSPG4-scFv-MK-6 was obtained by performing plasmid construction and IL-2 mutein production in the same manner as in Example 1 except for using DNA encoding an amino acid sequence set forth in SEQ ID NO: 20 or 19. Binding capability for each of human and mouse IL-2 receptor α-expressing 293T cells was similarly examined as a numerical value for an amount ratio (293Ta/293T). The results are shown in Fig. 11. As shown in Fig. 11, it is found that MSA-CSPG4-scFv-MK-14 bound to the human and mouse IL-2 receptor α, but as with MK-6, MSA-CSPG4-scFv-MK-6 does not bind to the human and mouse IL-2 receptor α.

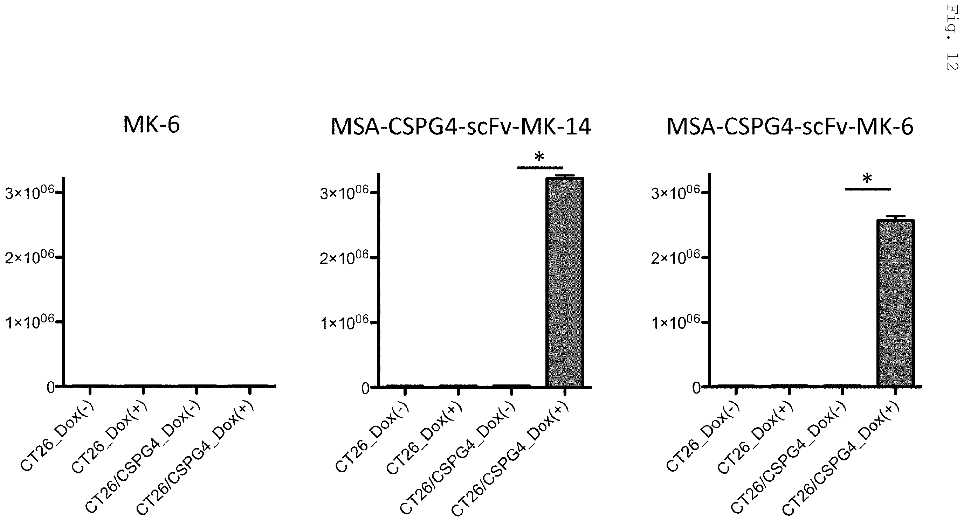

Next, binding to human CSPG4 on cells (on-cell receptor binding) was measured. Specifically, CT26 cells were purchased from the Cell Resource Center for Biomedical Research, Institute of Development, Aging and Cancer, Tohoku University. Human CSPG4-expressing CT26 cells (CT26/CSPG4) were established as CT26 cells inducibly expressing human CSPG4 in the presence of doxycycline through use of a lentiviral vector (LT3G-hCSPG4-myc-His, a plasmid obtained by inserting human CSPG4 gene cDNA into pmiRE18_LT3GEPIR_Ren713 provided by Dr. Johanness Zuber (Research Institute of Molecular Pathology (IMP))) constructed with human CSPG4 chain gene (NCBI Reference Sequence: NM_ 001897.5) as a cassette. CT26 and CT26/CSPG4 were allowed to adhere to a 24 wel cell culture plate (Techno Plastic Products AG, #92424), and then a suspension (10 nM) of NanoLuc (trademark) luciferase sequence (Promega Corporation, USA)-fused mature stable mutant human MK-6-IL-2 (MK-6, pcDNA4-MK-6-NLuc), mature stable mutant MSA-CSPG4-scFv-MK-6 (pcDNA4-MSA-CSPG4-scFv-MK-6-NLuc), or mature stable mutant MSA-CSPG4-scFv-MK-6 (pcDNA4-MSA-CSPG4-scFv-MK-6-NLuc) was added, and the mixture was subjected to a reaction by being left at rest at 4°C for 1 hour. The cells were washed, followed by quantification. The binding capability of each mutant IL-2 for human CSPG4 is shown as a numerical value for NanoLuc activity. The results are shown in Fig. 12. As shown in Fig. 12, MK-6 having albumin and CSPG-scFv fused thereto bound to CSPG4 as with MK-14 having albumin and CSPG-scFv fused thereto. It is found from the results that MK-6 having an antibody-like molecule conjugated thereto binds to the target antigen (human CSPG4) expressed on cancer cells.

### Example 2: IL-2-dependent Cell Proliferation

### Example 2-1: IL-2-dependent Cell Proliferation

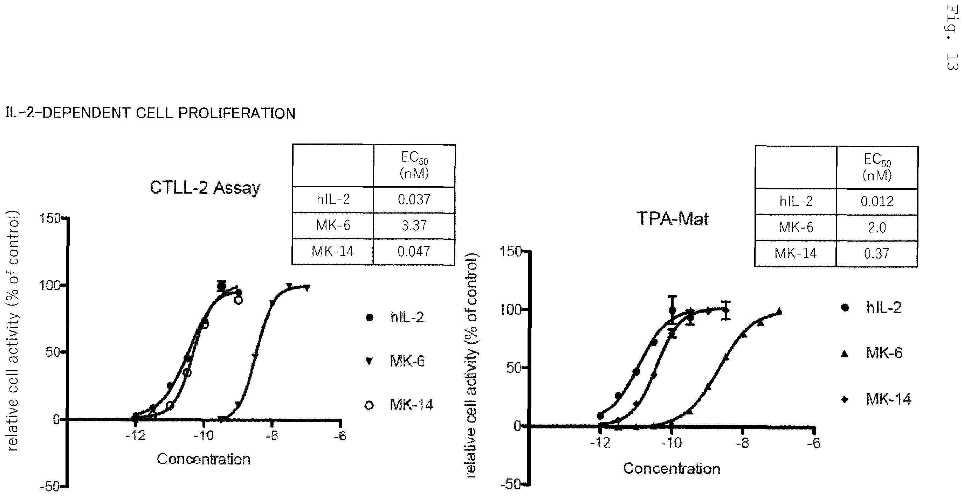

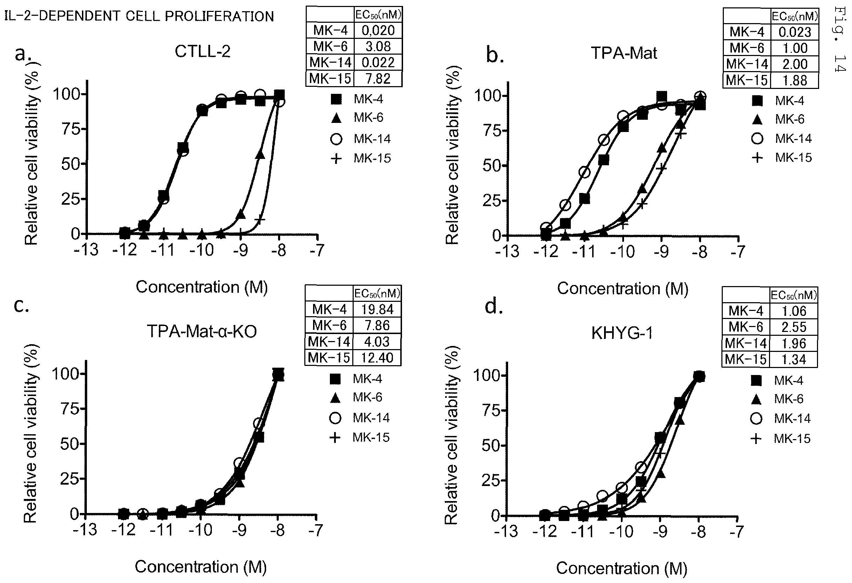

For proliferation of cultured cells with IL-2, CTLL-2 cells (RIKEN Cell Bank RCB0637), which were mouse T cell line CTLL-2 cells, were used. The cells proliferate in an IL-2-dependent manner, and hence are used for rapid and sensitive bioassay aimed at detecting IL-2. The CTLL-2 cells were grown at 37°C and 5% CO₂ or 37°C and 10% CO₂, and a humidity of 97%, using 1 nM human IL-2 (BioLegend, #589106), 50 µM 2-mercaptoethanol (Gibco (trademark)), 10% fetal bovine serum (FBS, Hyclone)-supplemented PRMI-1640 (FujiFilm-Wako, #18902025), 100 units/ml of penicillin, and 100 units/ml of streptomycin. CTLL-2 is a factor-dependent cell lineage that requires IL-2 for growth. Accordingly, in order to perform assay, the CTLL-2 cells were washed twice with high-glucose DMEM supplemented with 5% FBS, 5×10⁻⁵ M 2-mercaptoethanol, 50 µg/ml penicillin, 50 µg/ml streptomycin, and 3-4.0 mM L-glutamine to remove IL-2. An IL-2 sample to be assayed was titrated with 10% FBS-supplemented RPMI in a 96 wel cell culture plate (Techno Plastic Products AG, #92097). The washed CTLL-2 cells were added (final assay volume: 100 ul, 3,000 cells/well), and the plate was incubated for about 72 hours at 37°C and 10% CO₂. Proliferation of cultured human T cells with IL-2 was carried out using TPA-Mat cells (provided by Dr. Toshikazu Takeshita of Shinshu University. J Cell Physiol., 136: 319-25, 1988), which were an IL-2-dependent T cell line. The cells were grown in a medium (1 nM human IL-2 (BioLegend, #589106), 10% fetal bovine serum (FBS, Hyclone)-supplemented PRMI-1640 (FujiFilm-Wako, #18902025), penicillin: 100 units/ml, streptomycin: 100 units/ml) having human IL-2 added thereto at 37°C and 5% CO₂ or 37°C and 10% CO₂, and a humidity of 97%. Further, for the proliferation test of cultured human T cells with IL-2, cells expressing only the IL-2 receptor βγ-chains and not expressing the α-chain were also used. Specifically, KHYG-1 cells (provided by Dr. Toshikazu Takeshita of Shinshu University, expressing only the human IL-2 receptor βγ-chains and not expressing the α-chain) and TPA-Mat αKO cells (provided by Dr. Toshikazu Takeshita of Shinshu University. Expressing only the human IL-2 receptor βγ-chains and not expressing the α-chain), which were IL-2-dependent NK cell lines, were used. Those cells were washed twice, and then added to the IL-2 sample to be assayed. MK-4-IL-2 (MK-4), MK-6-IL-2 (MK-6), MK-14-IL-2 (MK-14), MK-15-IL-2 (MK-15), HSA-CSPG4-scFv-MK-14, or HSA-CSPG4-scFv-MK-6 was used as the IL-2 sample to be assayed. HSA-CSPG4-scFv-MK-14 or HSA-CSPG4-scFv-MK-6 was obtained by performing plasmid construction and IL-2 mutein production in the same manner as in Example 1 except for using DNA encoding an amino acid sequence set forth in SEQ ID NO: 17 or 16. Those IL-2 samples were titrated with 10% FBS-supplemented RPMI1640 in a 96 wel cell culture plate (Techno Plastic Products AG, #) and prepared as samples having increasing concentrations. The washed TPA-Mat, or TPA-Mat αKO cells or KHYG-1 cells were added (final assay volume: 100 ul, 3,000 cells/well), and the plate was incubated for about 72 hours at 37°C and 10% CO₂. The cell count of the cells of each well was quantitatively measured using CellTiter-Glo (trademark) Luminescent Cell Viability Assay Kit (Promega Corporation, #G7570), and was expressed as a relative value with the Emax of each IL-2 (including the mutants) being defined as 100%. The results for IL-2, MK-6, and MK-14 are shown in Fig. 13. The results for MK-6, MK-4, MK-14, and MK-15 are shown in Fig. 14. The "IL-2-dependent proliferation" is a numerical value related to biological activity, and refers to a measured value for biological activity in quantitative in vitro assay based on cells. As shown in Fig. 13, in the proliferation test using CTLL-2, the 50% effective concentration (EC50) of MK-6-IL-2 was decreased as compared to the 50% effective concentration (EC50) of each of MK-14-IL-2 and IL-2 (BioLegend, #589106). Likewise, also in the analysis using TPA-Mat, results similar to those in the case of CTLL-2 were obtained. Thus, MK-6 exhibited comparable maximum proliferation effects on the mouse and human T cell lines to those of IL-2 and MK-14-IL-2. Further, as shown in Fig. 14, MK-15 showed proliferation activity on each of CTLL-2 and TPA-Mat as with MK-6. Further, MK-6 and MK-15 each exhibited proliferation activity on the TPA-Mat αKO cells or the KHYG-1 cells. Because of showing proliferation activity comparable to that of MK-14 on those cells expressing only the IL-2 receptor βγ-chains and not expressing the α-chain, MK-6 and MK-15 are each found to have an ability to exhibit proliferation activity similar to that of IL-2 on human IL-2 receptor βγ-chain-expressing cells.

### Example 2-2

A proliferation test using CTLL-2 was performed in the same manner as in Example 2-1 except for using MK-6-IL-2, HSA-3×G4S-MK-6, or HSA-3×(EAAAK)-MK-6 as an IL-2 sample. HSA-3xG4S-MK-6 or HSA-3×(EAAAK)-MK-6 is a product obtained by changing the linker sequence of MK-6-IL-2 to 3×G4S or 3×(EAAAK), respectively, and the amino acid sequence thereof is set forth in SEQ ID NO: 7 or 9. Those IL-2 samples were also obtained by performing plasmid construction and IL-2 mutein production in the same manner as in Example 1 except for using DNA encoding the amino acid sequence set forth in SEQ ID NO: 7 or 9.

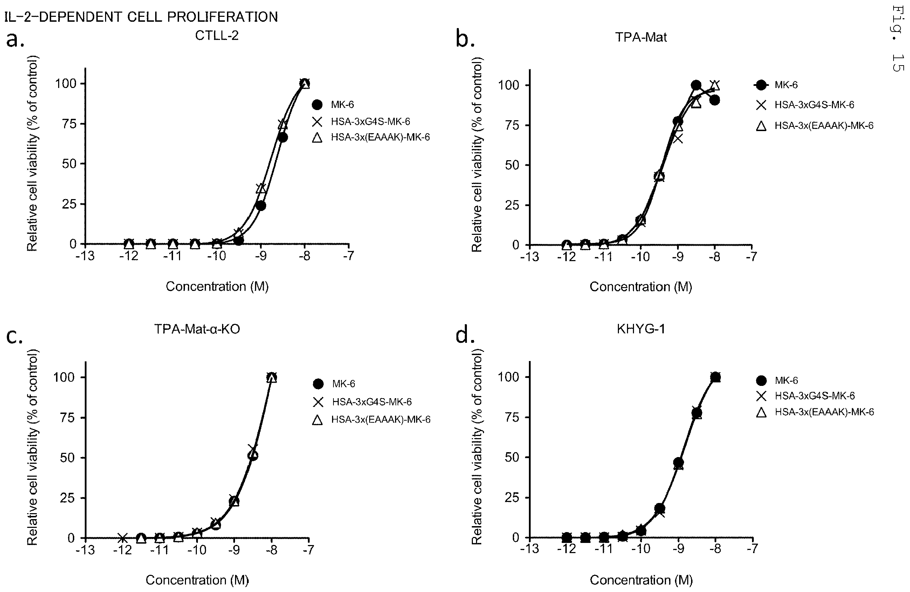

As shown in Fig. 15, in the proliferation test using CTLL-2, MK-6-IL-2, HSA-3×G4S-MK-6, and HSA-3×(EAAAK)-MK-6 all showed proliferation activities comparable to each other. Similarly, also in the analysis using TPA-Mat, TPA-Mat αKO cells, or KHYG-1 cells, results similar to those in the case of CTLL-2 were obtained. Thus, albumin-fused MK-6-IL-2 exhibited comparable proliferation effects on the mouse and human T cell lines to those of MK-6-IL-2, whether a flexible linker was used or a rigid linker was used.

### Example 2-3

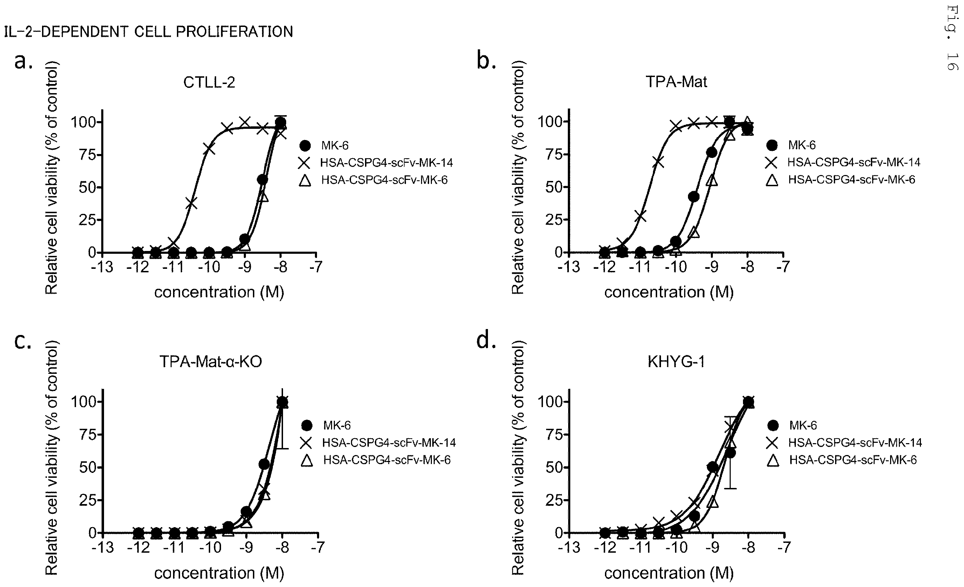

An IL-2-dependent proliferation test was performed in the same manner as in Example 2-1 except for using MK-6-IL-2, HSA-CSPG4-scFv-MK-6, and HSA-CSPG4-scFv-MK-14 as IL-2 samples. The results are shown in Fig. 16. As shown in Fig. 16, HSA-CSPG4-scFv-MK-14 showed high proliferation activity in terms of proliferation activity on each of CTLL-2 and TPA-Mat. Meanwhile, HSA-CSPG4-scFv-MK-6 showed proliferation activity comparable to that of MK-6-IL-2. HSA-CSPG4-scFv-MK-6 exhibited comparable proliferation activity on the TPA-Mat αKO cells or the KHYG-1 cells to those of HSA-CSPG4-scFv-MK-14 and MK-6-IL-2. The fact that the three showed proliferation activities comparable to each other on those cells expressing only the IL-2 receptor βγ-chains and not expressing the α-chain indicates that, even when HSA and CSPG4 are linked to MK-6 via the G4S linker, the resultant has an ability to exhibit similar proliferation activity on human IL-2 receptor βγ-chain-expressing cells.

### Example 3: IL-2-dependent Treg Cell Proliferation

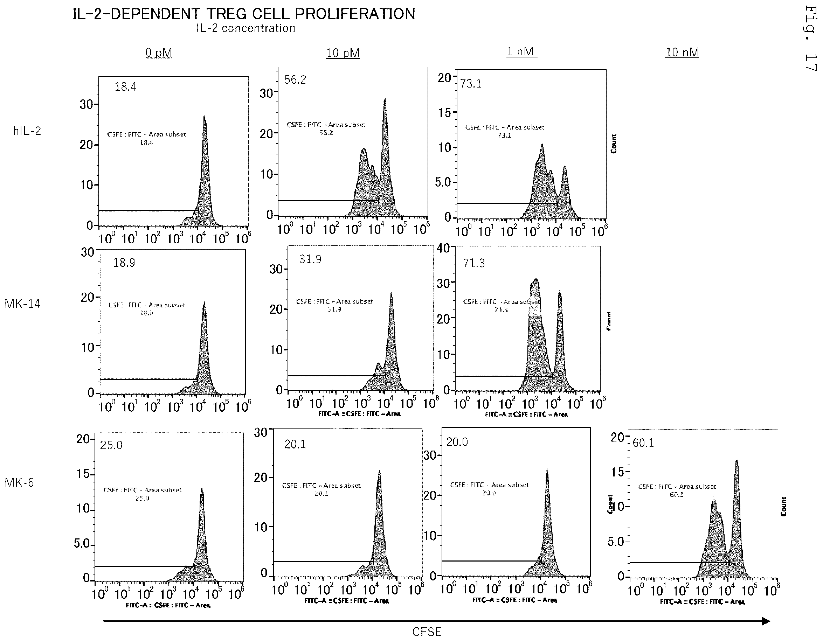

Treg cells (CD4⁺CD25⁺) were prepared from the spleen of BALB/c mice (8-week-old, Japan SLC, Inc.) through use of CD4⁺CD25⁺ Regulatory T cell Isolation Kit (Miltenyi Biotec, #130-091-041) in accordance with the manufacturer's instructions. Next, the Treg cells were washed twice with 1×D-PBS(-) (Gibco (trademark)), and then resuspended in 1×D-PBS(-), followed by staining with 5,6-carboxyfluorescein diacetate succinimidyl ester (CFSE, BioLegend, #423801) at room temperature and in a dark place for 15 minutes. 10% Fetal bovine serum (FBS, Hyclone)-supplemented PRMI-1640 (FujiFilm-Wako, 18902025)/supplemented with 50 µM 2-mercaptoethanol (Gibco (trademark))/100 units/ml of penicillin/100 units/ml of streptomycin) was added to terminate the reaction, and the cells were suspended again in 10% fetal bovine serum (FBS, Hyclone)-supplemented PRMI-1640 liquid. A 96 wel cell culture plate (Techno Plastic Products AG, #92097), which had in advance been coated with 1×D-PBS(-) (Gibco (trademark)) having added thereto an anti-CD3 monoclonal antibody (1 µg/mL, BioLegend, Purified anti-mouse CD3ε Antibody #100301) at 4°C for 16 hours and washed 3 times with 1×D-PBS(-) (Gibco (trademark)), was used and loaded with 100,000 Treg cells per well, an anti-CD28 antibody Purified anti-human CD28 Antibody (BioLegend, #102112), and IL-2 and MK-14-IL-2, which had been titrated to have increasing concentrations, and the cells were cultured at 37°C, 5% CO₂, and a humidity of 97%. After 72 hours, the cells were recovered, and CSFE stainability was quantitatively analyzed by flow cytometry (FACS). The results are shown in Fig. 17. Cell proliferation is indicated by the CSFE stainability, which decreases in a manner dependent on the number of cell divisions. As shown in Fig. 17, IL-2 (BioLegend, #589106) and MK-14-IL-2 induced cell division and proliferation for more than 70% of Tregs at a concentration of 1 nM, but MK-6 showed no change in cell division-increasing action on Tregs as compared to the case of no addition (0 pM), and hence lacked proliferation activity on Tregs.

### Example 4: Effects of IL-2 In Vivo Injection on Peripheral Immune Cells

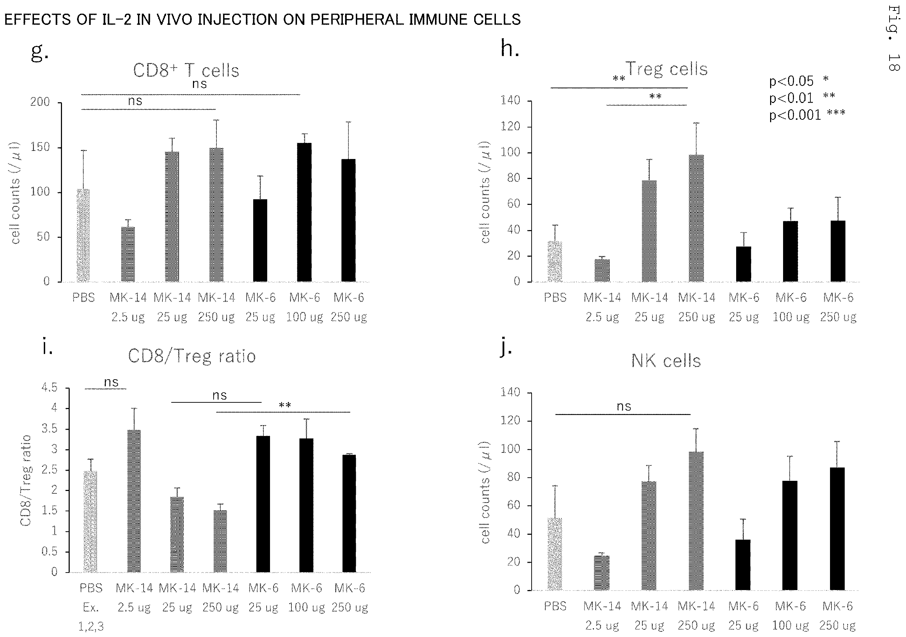

Effects on peripheral immune system cell counts in mice (BALB/c) injected with IL-2 (MK-6-IL-2 or MK-14-IL-2) were measured. Specifically, BALB/c mice (8-week-old, n=3) purchased from Japan SLC, Inc. were intravenously injected with IL-2 in amounts of 2.5 µg, 25 µg, and 250 µg per individual once daily for 4 consecutive days, and peripheral blood immune system cells were prepared from the mouse spleen the day after the final injection. CD8⁺ T cells (CD3⁺CD4⁻CD8⁺:), Treg cells (CD3⁺CD4⁺CD25⁺FoxP3⁺), and NK (CD3-CD49b⁺NKp46⁺) were stained with antibodies, and their cell counts were quantified by FACS analysis. The antibodies used are as follows: CD3ε: 145-2C11, BioLegend, CD4: RM4-4, BioLegend, CD8a: 53-6.7, BioLegend, CD25 (IL-2Rα): PC61, BioLegend, NKp46: 29A1.4, BioLegend, and FoxP3: FJK-16s, DB Biosciences. The results are shown in Fig. 18. As shown in Fig. 18, none of MK-14-IL-2 and MK-6 had an ability to increase intratumoral CD8⁺ T cells and NK cells. Meanwhile, MK-14-IL-2 increased Treg cells by 25 µg injection, but MK-6 did not increase the cells even by 250 µg injection. Further, a CD8/Treg ratio was not increased by MK-14-IL-2 as compared to the PBS-injected group, but was significantly increased by 250 µg injection in the case of MK-6 injection as compared to MK-14-IL-2.

### Example 5: Side Effects of IL-2 In Vivo Injection

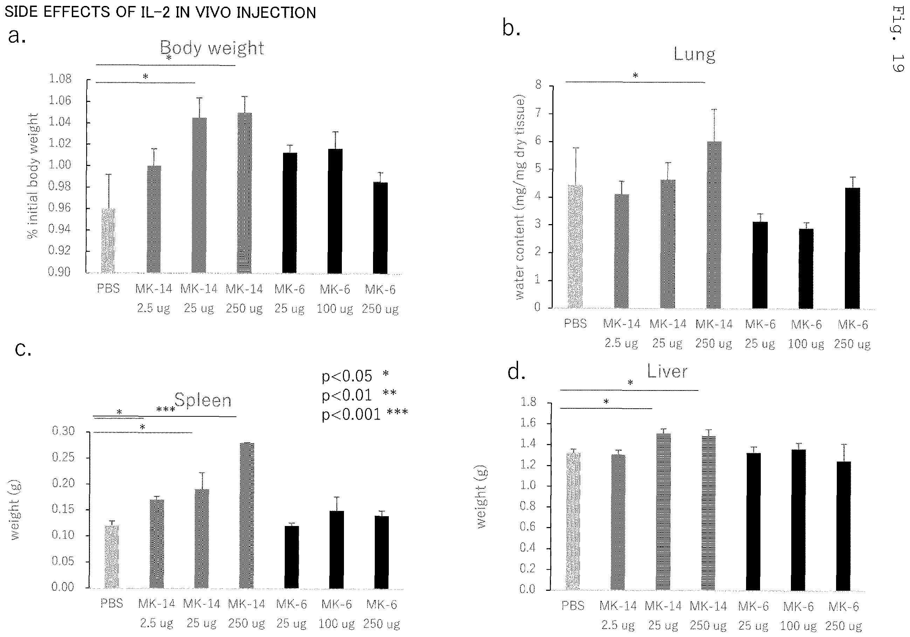

IL-2 (MK-6-IL-2 or MK-14-IL-2) was injected into mice (BALB/c, 8-week-old, Japan SLC, Inc.), and the in vivo action thereof was investigated. Specifically, each IL-2 was intravenously injected in amounts of 2.5 gg, 25 gg, and 250 µg once daily for 4 consecutive days. The mice were euthanized on day 5. Body weight measurement was performed, and the ratio of increase in body weight as compared to that before the injection was calculated. In addition, the liver and the spleen were excised, and their respective weights were measured. The lung was subjected to weight measurement after excision, and then dried using miVac (Genevac, SP Scientific) and measured for a dry weight, followed by calculation of a water content by dividing the latter by the former. The side effects of IL-2 injection are shown as increases in body weight and weight of each organ. The results are shown in Fig. 19. Specifically, MK-14-IL-2 significantly increased the body weight by 25 µg injection, but MK-6 did not increase the body weight even by 250 µg injection. For each of the spleen and the liver, 25 µg of MK-14-IL-2 significantly increased the weight of the organ, but MK-6 did not show a similar action. The occurrence of pulmonary edema is shown as a value obtained by dividing the lung weight by the dry weight (water content value per dry weight). Pulmonary edema occurred through 250 µg injection of MK-14-IL-2, but no pulmonary edema occurred through injection of the same volume in the case of MK-6 injection.

### Example 6: Antitumor Effect of IL-2 Monotherapy in CT26 Colon Cancer-bearing Mouse Model

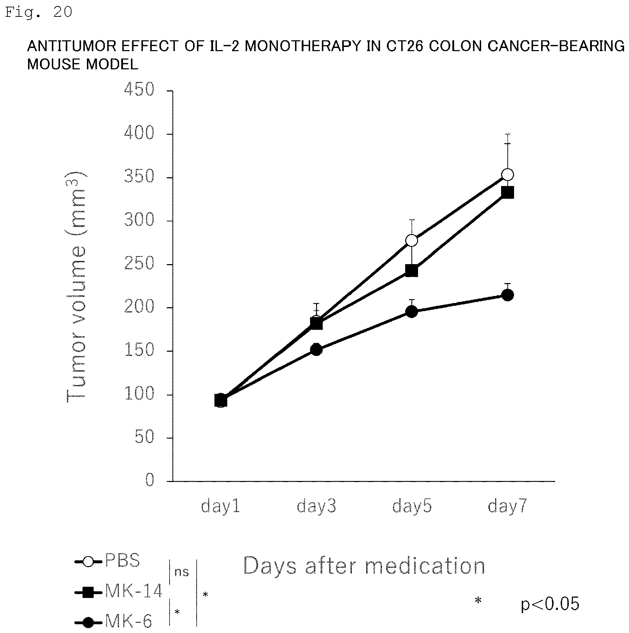

The effect of IL-2 (MK-14-IL-2 or MK-6-IL-2) on cancer-bearing mice (BALB/c) subcutaneously implanted with CT26 colon cancer cells was measured. Specifically, 5×10⁵ CT26 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of mice of the same species as the cells (BALB/c). 1×D-PBS(-), MK-14-IL-2 (5 µg), or MK-6-IL-2 (100 µg) was intraperitoneally injected twice daily for 5 consecutive days, and tumor diameters (shortest diameter "a" mm and longest diameter "b" mm) were measured over time using a caliper. A tumor volume (V) was calculated using the approximate formula: longest diameter×longest diameter×shortest diameter÷2 (V=a×b²/2)mm³. The results of the measurement of the tumor system are shown in Fig. 20. As shown in Fig. 20, the groups injected with 1×D-PBS(-) and MK-14-IL-2 showed similar growths of tumors, but tumor proliferation was significantly suppressed in the group injected with MK-6-IL-2 as compared to the MK-14-IL-2 group.

### Example 7: FACS Analysis of Intratumoral T Cells through IL-2 Monotherapy in CT26 Colon Cancer-bearing Mouse Model

Intratumoral T cells through IL-2 monotherapy in a CT26 colon cancer-bearing mouse model were prepared, and were subjected to FACS analysis. Specifically, 5×10⁵ CT26 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of BALB/c mice (8-week-old, Japan SLC, Inc.) using a 26G needle and a 1 mL syringe. Tumors that had grown to a uniform size were intraperitoneally injected with 1×D-PBS(-) or IL-2 (MK-14-IL-2 or MK-6-IL-2) twice daily for 5 consecutive days, and then intratumoral lymphocytes (TILs) were prepared from the excised tumors. Specifically, the excised tumors were minced with scissors and the like, and then immersed in 10 mL of dissociation buffer (10% fetal bovine serum (FBS, Hyclone)-supplemented PRMI-1640 (FujiFilm-Wako, 18902025)/penicillin: 100 units/ml/streptomycin: 100 units/ml, DNaseI (Sigma-Aldrich #DN25-100MG, added at 50 µg/mL) and shaken at 37°C and 122 rpm for 30 minutes (Taitec Corporation, BR-53FP). The cell suspension was passed through a 70 µm strainer (BD Falcon) to be turned into single cells, and then hematopoietic cells (CD45+) were prepared as TILs with MACS Pro Separator (Miltenyi Biotec) through use of TIL (CD45) MicroBeads, mouse (Miltenyi Biotec, #130-110-618).

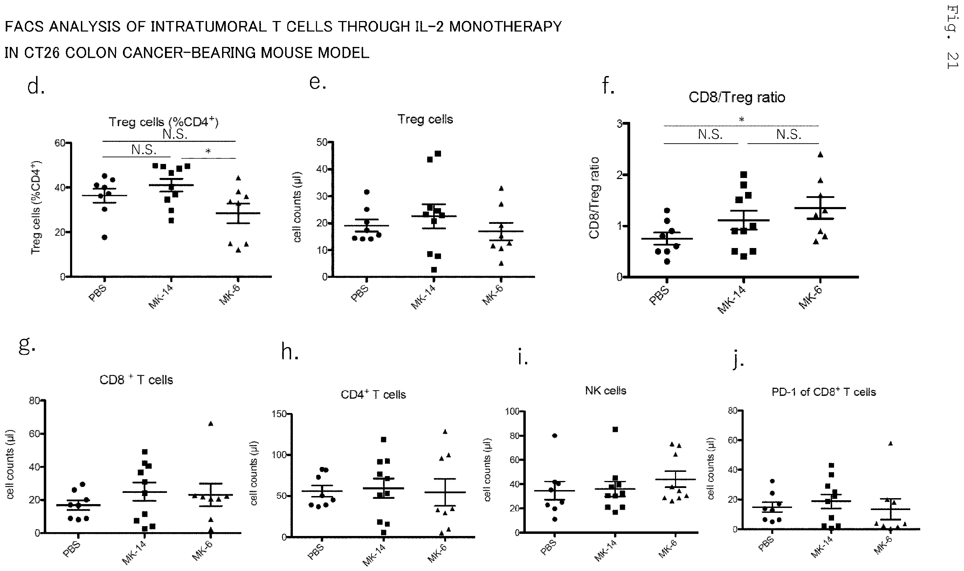

The results of FACS analysis of the prepared TILs are shown in Figs. 21. Treg cells (CD4⁺CD25⁺FoxP3⁺), CD8⁺ T cells, PD-1⁺CD8⁺ T cells, CD4⁺ T cells, and NK cells (CD3-CD49b⁺NKp46⁺) were analyzed by FACS, and their respective cell counts were quantified. A CD8/Treg ratio is shown as a relative value obtained by dividing the CD8⁺ T cell count by the Treg cell count. The antibodies used are as follows: CD3ε: 145-2C11, BioLegend, CD4: RM4-4, BioLegend, CD8a: 53-6.7, BioLegend, CD25 (IL-2Rα): PC61, BioLegend, NKp46: 29A1.4, BioLegend, FoxP3: FJK-16s, DB Biosciences, and PD-1: 29F.1A12, BioLegend. As shown in Figs. 21, the TILs derived from the mice injected with MK-6-IL-2 had significantly fewer Tregs (%) in the CD4+ T cell group as compared to the group injected with MK-14-IL-2 (Fig. 21d). Further, the CD8/Treg ratio was significantly increased as compared to the PBS-injected group. Meanwhile, in this tumor model, no differences were found for CD4⁺ T cells, CD8⁺ T cells, PD-1⁺CD8⁺ T cells, and NK cells.

### Example 8: Antitumor Effect of IL-2 Monotherapy in B16F10 Melanoma-bearing Mouse Model

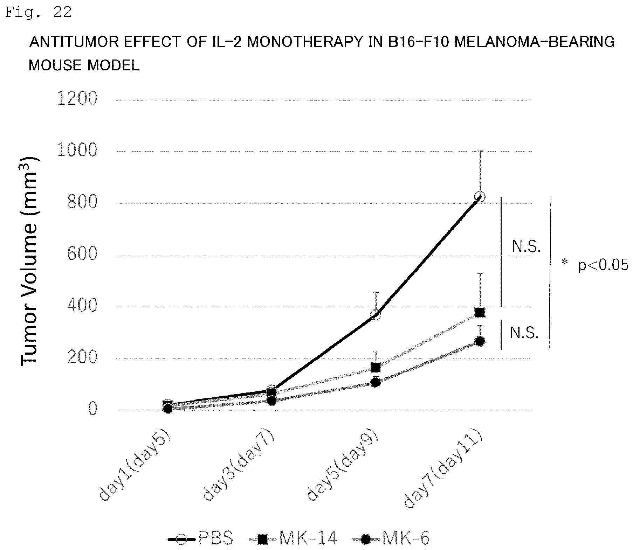

The effect of IL-2 (MK-6-IL-2 or MK-14-IL-2) on cancer-bearing mice (C57BL/6) subcutaneously implanted with B16F10 melanoma was measured. Specifically, 5×10⁵ B16-F10 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of C57/BL6 mice (8-week-old, Japan SLC, Inc.)) through use of a 26G needle and a 1 mL syringe. Tumors that had grown to a uniform size were intraperitoneally injected with 1×D-PBS(-), MK-14-IL-2 (5 µg), or MK-6-IL-2 (100 µg) twice daily for 5 consecutive days, and tumor diameters (shortest diameter "a" mm and longest diameter "b" mm) were measured over time using a caliper. A tumor volume (V) was calculated using the approximate formula: longest diameter×longest diameter×shortest diameter÷2 (V=a×b²/2)mm³. The results of the measurement of the tumor system are shown in Fig. 22. As shown in Fig. 22, MK-6 showed a significantly smaller tumor volume than 1×D-PBS(-) on day 7 after the start of IL-2 injection.

### Example 9: FACS Analysis of Intratumoral Lymphocytes through IL-2 Monotherapy in B16F10 Melanoma-bearing Mouse Model

FACS analysis of intratumoral T cells through IL-2 monotherapy in a B16F10 melanoma-bearing mouse model was performed. Specifically, 5×10⁵ B16F10 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of C57/BL6 mice (8-week-old, Japan SLC, Inc.)) through use of a 26G needle and a 1 mL syringe. Tumors that had grown to a uniform size were intraperitoneally injected with 1×D-PBS(-), MK-14-IL-2 (5 µg), or MK-6-IL-2 (100 µg) twice daily for 5 consecutive days, and then intratumoral lymphocytes (TILs) were prepared from the excised tumors. Specifically, the excised tumors were minced with scissors and the like, and then immersed in 10 mL of dissociation buffer (10% fetal bovine serum (FBS, Hyclone)-supplemented PRMI-1640 (FujiFilm-Wako, #18902025)/penicillin: 100 units/ml/streptomycin: 100 units/ml, DNaseI (Sigma-Aldrich, #DN25-100MG, added at 50 µg/mL) and shaken at 37°C and 122 rpm for 30 minutes (Taitec Corporation, BR-53FP). The cell suspension was passed through a 70 µm strainer (BD Falcon) to be turned into single cells, and then hematopoietic cells (CD45+) were prepared as TILs with MACS Pro Separator (Miltenyi Biotec) through use of TIL (CD45) MicroBeads, mouse (Miltenyi Biotec, #130-110-618).

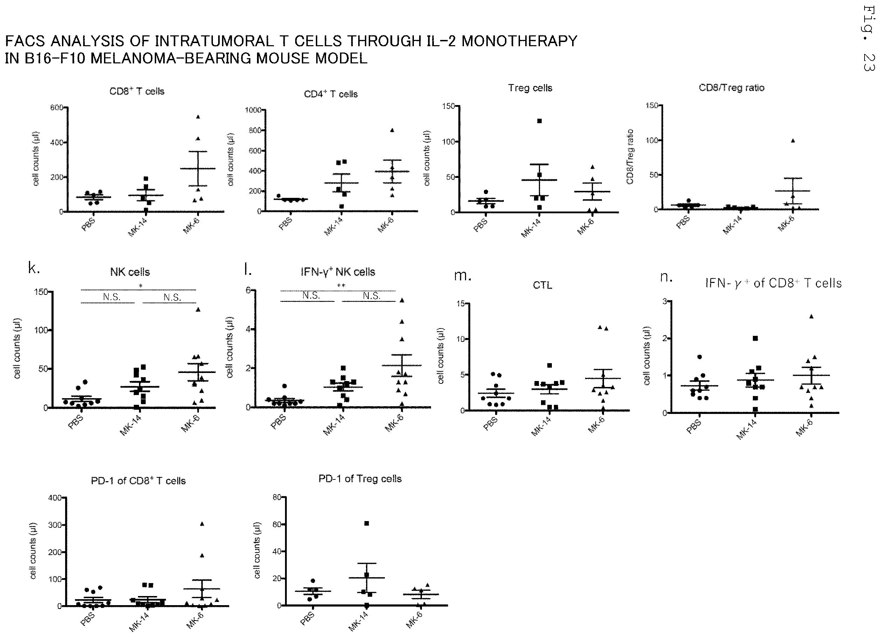

For the results of FACS analysis of the prepared TILs, FACS analysis of the excised intratumoral lymphocytes (TILs) was performed. The respective cell counts of CD8⁺ T cells, CD4⁺ T cells, Treg cells (CD4⁺CD25⁺FoxP3⁺), IFNγ⁺CD8⁺ T cells (activated CD8⁺ T cells) NK cells (CD3⁻CD49b⁺NKp46⁺), IFNγ⁺ NK cells (activated NK cells), PD-1⁺CD8⁺ T cells, and PD-1⁺ Treg cells (activated Treg cells) were quantified. The antibodies used are as follows: CD3ε: 145-2C11, BioLegend, CD4: RM4-4, BioLegend, CD8a: 53-6.7, BioLegend, CD25 (IL-2Rα): PC61, BioLegend, NKp46: 29A1.4, BioLegend, FoxP3: FJK-16s, DB Biosciences, PD-1: 29F.1A12, BioLegend, and IFNγ: 4SB3, BioLegend. A CD8/Treg ratio is shown as a relative value obtained by dividing the CD8⁺ T cell count by the Treg cell count. The results are shown in Fig. 23. As shown in Fig. 23, there were no significant differences for CD8⁺ T cells, CD4⁺ T cells, Treg cells (CD4⁺CD25⁺FoxP3⁺), and IFNγ⁺CD8⁺ T cells (activated CD8⁺ T cells), but NK cells (CD3-CD49b⁺NKp46⁺) and IFNγ⁺NK cells each had a significantly higher count in the MK-6-IL-2-injected group than in the PBS-injected group.

### Example 10: Antitumor Effect of Mature MK-6-IL-2-Mouse Albumin (SEQ ID NO: 12) Monotherapy in CT26 Colon Cancer-bearing Mouse Model

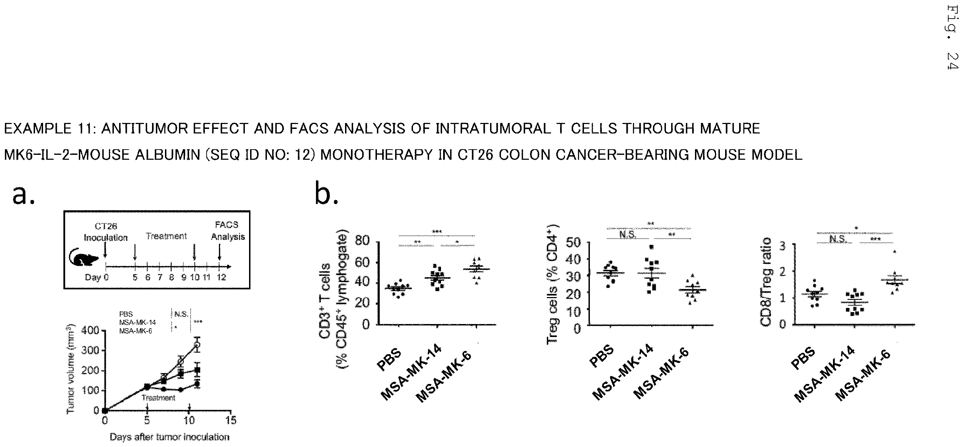

The effect of MSA-IL-2 (MSA-MK-14-IL-2 (SEQ ID NO: 13) or MSA-MK-6-IL-2 (SEQ ID NO: 12)) on cancer-bearing mice (BALB/c) subcutaneously implanted with CT26 colon cancer cells was measured. Specifically, 5×10⁵ CT26 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of mice of the same species as the cells (BALB/c). 1×D-PBS(-), MSA-MK-14-IL-2 (5 µg), or MSA-MK-6-IL-2 (100 µg) was intraperitoneally injected twice in total on day 5 and day 10, and tumor diameters (shortest diameter "a" mm and longest diameter "b" mm) were measured over time using a caliper. A tumor volume (V) was calculated using the approximate formula: longest diameter×longest diameter×shortest diameter÷2 (V=a×b²/2)mm³. The results of the measurement of the tumor system are shown in Fig. 24a. As shown in Fig. 24a, the tumor injected with 1×D-PBS(-) grew over time, but the group injected with MSA-MK-14-IL-2 had no significant difference in tumor-shrinking effect. Meanwhile, in the group injected with MSA-MK-6-IL-2, tumor proliferation was significantly suppressed as compared to each of the PBS and MSA-MK-14-IL-2 groups.

### Example 11: FACS Analysis of Intratumoral T Cells through Mature MK-6-IL-2-mouse Albumin (SEQ ID NO: 12) Monotherapy in CT26 Colon Cancer-bearing Mouse Model:

Intratumoral T cells through IL-2 monotherapy in a CT26 colon cancer-bearing mouse model were prepared, and were subjected to FACS analysis. Specifically, 5×10⁵ CT26 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of BALB/c mice (8-week-old, Japan SLC, Inc.) through use of a 26G needle and a 1 mL syringe. Tumors that had grown to a uniform size were intraperitoneally injected with 1×D-PBS(-) or albumin-fused IL-2 (MSA-MK-14-IL-2 or MSA-MK-6-IL-2) twice in total on day 5 and day 10, and intratumoral lymphocytes (TILs) were prepared from the tumors excised on day 12. Specifically, the excised tumors were minced with scissors and the like, and then immersed in 10 mL of dissociation buffer (10% fetal bovine serum (FBS, Hyclone)-supplemented PRMI-1640 (FujiFilm-Wako, 18902025)/penicillin: 100 units/ml/streptomycin: 100 units/ml, DNaseI (Sigma-Aldrich, #DN25-100MG, added at 50 µg/mL) and shaken at 37°C and 122 rpm for 30 minutes (Taitec Corporation, BR-53FP). The cell suspension was passed through a 70 µm strainer (BD Falcon) to be turned into single cells, and then hematopoietic cells (CD45+) were prepared as TILs with MACS Pro Separator (Miltenyi Biotec) through use of TIL (CD45) MicroBeads, mouse (Miltenyi Biotec, #130-110-618). The results of FACS analysis of the prepared TILs are shown in Fig. 16. Treg cells (CD4⁺CD25⁺FoxP3⁺), CD8⁺ T cells, PD-1⁺CD8⁺ T cells, CD4⁺ T cells, and NK cells (CD3-CD49b⁺NKp46⁺) were analyzed by FACS, and their respective cell counts were quantified. A CD8/Treg ratio is shown as a relative value obtained by dividing the CD8⁺ T cell count by the Treg cell count. The antibodies used are as follows: CD3ε: 145-2C11, BioLegend, CD4: RM4-4, BioLegend, CD8a: 53-6.7, BioLegend, CD25 (IL-2Rα): PC61, BioLegend, NKp46: 29A1.4, BioLegend, FoxP3: FJK-16s, DB Biosciences, and PD-1: 29F.1A12, BioLegend. The results are shown in Fig. 24b. As shown in Fig. 24b, the TILs derived from the mice injected with mature MK-6-IL-2-mouse albumin (SEQ ID NO: 12) had a significantly high ratio of the CD3+ T cell group and significantly fewer Tregs (%) in the CD4+ T cell group as compared to the group injected with mature MK-14-IL-2-mouse albumin (SEQ ID NO: 13) (Fig. 24b). Further, the CD8/Treg ratio was significantly increased as compared to the PBS-injected group and the mature MK-14-IL-2-mouse albumin-injected group.

### Example 12: Changes in IL-2 Blood Concentration (a) after Intraperitoneal Injection of Mature MK-6-IL-2 (SEQ ID NO: 3) and Mature MK-6-IL-2-mouse Albumin (SEQ ID NO: 12) and Quantification of Intratumoral IL-2 in CT26 Colon Cancer-bearing Mouse Model

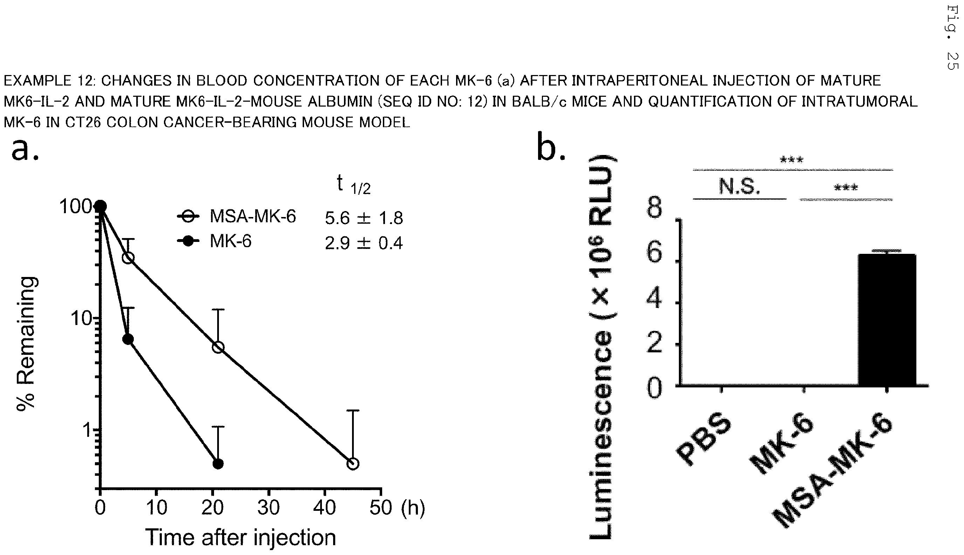

(a) BALB/c mice (8-week-old, Japan SLC, Inc.) were intraperitoneally injected with MK-6 (SEQ ID NO: 3) (25 mg) and MSA-MK-6 (SEQ ID NO: 12) (65 mg) corresponding to equimolar amounts. Each IL-2 has fused thereto a NanoLuc (trademark) luciferase sequence (Promega Corporation, USA). Blood IL-2 after the injection was quantified based on NanoLuc (trademark) activity. Half-lives in a beta-phase (β-phase) starting from 1 hour after the injection (Mean β half-lives (t_{1/2}))±standard deviation (SD) were measured. "%Remaining" is shown as a relative value (%) obtained by dividing each measured value for NanoLuc activity at 1 hour after the injection by a measured value for NanoLuc activity at each time point. The "β-phase" refers to a phase (β-phase) in which subsequent blood concentration changes show slow attenuation following an initial phase (α-phase) of rapid attenuation, and the half-lives are calculated in this phase. The results are shown in Fig. 24a. As shown in Fig. 24a, mature MK-6-IL-2-mouse albumin having fused thereto blood MSA after medication had a t_{1/2} of 5.6 hours, the blood half-life being much longer as compared to mature MK-6-IL-2 having a t_{1/2} of 2.9 hours. (b) Quantification of intratumoral IL-2 in CT26 colon cancer-bearing mouse model: 5×10⁵ CT26 cells (purchased from the Cell Resource Center for Biomedical Research, Institute of Development, Aging and Cancer, Tohoku University) were suspended in 100 µL of 1×D-PBS(-), and the suspension was subcutaneously implanted into the back of BALB/c mice (8-week-old, Japan SLC, Inc.) through use of a 26G needle and a 1 mL syringe. Tumors that had grown to a uniform size were intraperitoneally injected with mature MK-6-IL-2 (SEQ ID NO: 3) and mature MK-6-IL-2-mouse albumin (SEQ ID NO: 12) only once. After 1 hour from the injection, the tumors were excised, and 1×D-PBS(-) was added, followed by crushing of the tumors with BioMasher (trademark, Nippi, Incorporated). The NanoLuc activity was quantified using Lumat³ LB9508 from Berthold Japan K.K. with a specimen prepared using Nano-Glo (trademark) Luciferase Assay System from Promega K.K. The results are shown in Fig. 25b. As shown in Fig. 25b, it was shown that mature MSA-MK-6-IL-2 was accumulated in the tumor at a significantly high concentration.

## Claims

1. An IL-2 mutein, comprising:
an amino acid sequence set forth in SEQ ID NO: 3; or
an amino acid sequence which has at least 95% sequence identity to the amino acid sequence of SEQ ID NO: 3 and in which amino acids at position 35, position 38, position 42 or position 43, and position 45 are all A's.

2. The IL-2 mutein according to claim 1, wherein the IL-2 mutein is a fusion protein with albumin and/or an immunoglobulin.

3. The IL-2 mutein according to claim 2, wherein an immunoglobulin portion of the fusion protein is an Fc portion of an antibody.

4. The IL-2 mutein according to claim 2, wherein the IL-2 mutein is albumin-IL-2, IL-2-albumin, albumin-IL-2-immunoglobulin, immunoglobulin-IL-2-albumin, albumin-immunoglobulin-IL-2-albumin, or albumin-IL-2-immunoglobulin-albumin.

5. The IL-2 mutein according to claim 1, wherein the IL-2 mutein has an ability to activate cells expressing IL-2 receptors β and γ, and has reduced binding capability for an IL-2 receptor α as compared to an IL-2 mutein formed of an amino acid sequence set forth in SEQ ID NO: 2.

6. A pharmaceutical, comprising the IL-2 mutein of claim 1.

7. The pharmaceutical according to claim 6, wherein the pharmaceutical is for preventing or treating cancer.
